# EUROPEAN PATENT APPLICATION

(11) **EP 4 814 982 A1**
(43) Date of publication of application: **30.09.2026**
(21) Application number: 24894618.8
(22) Date of filing: 20.11.2024
(51) Int. Cl.: G16H 50/20, G16H 50/70, G16B 25/10, G16B 20/20, G16B 40/20, G06N 20/00

(54) **METHOD FOR PROVIDING CANCER RISK INFORMATION USING COX REGRESSION ANALYSIS ALGORITHM**

(30) Priority: 20.11.2023 KR 20230160497
(71) Applicant: DCGen Co., Ltd., Seoul 03170 (KR)
(72) Inventor: MOON, Kyung Chul, Seoul 03080 (KR); JEONG, Chang Wook, Seoul 03080 (KR); HAN, Jang Hee, Seoul 03080 (KR); CHO, Nam Hoon, Seoul 03722 (KR); CHOI, Young Deuk, Seoul 03722 (KR); HAN, Hyun Ho, Seoul 03722 (KR); CHUNG, Woo Sung, Seoul 03170 (KR); LEE, Jong Keun, Seoul 03170 (KR); AHN, Ji Young, Seoul 03170 (KR)
(74) Representative: Mewburn Ellis LLP
(86) International application number: PCT/KR2024/018426
(87) International publication number: WO 2025/110726

(57) **Abstract**

The present invention relates to a method for providing information regarding cancer risk. More specifically, the present invention applies a Cox regression analysis algorithm to a combination of biomarkers that enables the analysis of prostate cancer metastasis and allows for more precise diagnosis, thereby providing personalized information regarding a patient's cancer risk when determining a treatment method.

## Description

### Technical Field

The present invention relates to a method for predicting cancer risk by applying a Cox regression analysis algorithm.

### Background Art

Cancer is one of the most common causes of death worldwide. Approximately 10 million new cases occur annually, accounting for about 12% of all deaths and making it the third leading cause of death. Among the various types of cancer, prostate cancer is the most common cancer among men worldwide and ranks second in mortality. It typically affects men over the age of 50, and the number of patients increases sharply with age. Although it usually progresses slowly, it becomes extremely difficult to treat once it becomes malignant and metastasizes. Metastasis typically begins in the lymph nodes surrounding the prostate, the pelvic bones, the spine, and the bladder, and gradually spreads throughout the body.

Current diagnostic methods for prostate cancer primarily include the prostate-specific antigen (PSA) test and digital rectal examination (DRE). Imaging methods such as transrectal ultrasound, CT, MRI, and whole-body bone scans (WBBS) are also used, and biopsies are performed. However, in most cases, these methods have low diagnostic accuracy, make early diagnosis difficult, and do not clearly distinguish between benign conditions such as benign prostatic hyperplasia and prostatitis, making it challenging to diagnose prostate cancer based on its specific type.

Although prognostic prediction tools based on RNA expression levels (such as Decipher, Prolaris, and Oncotype DX) have recently entered the market, they fall under technologies that utilize classical methods such as PCR and DNA microarrays, and the number of markers that can be predicted simultaneously is limited to around 30. Furthermore, the prognostic accuracy of factors currently used for prognosis prediction-including prostate cancer staging (TNM stage)-is significantly lower compared to other cancer types. Moreover, there is a lack of tools capable of precisely predicting metastasis and recurrence within the realm of prognosis prediction.

Accordingly, the inventors of the present invention have identified a combination of biomarkers capable of analyzing the presence of metastasis in prostate cancer and enabling more precise diagnosis. By applying these combination markers to a Cox regression analysis algorithm, they aim to provide information on individual patient cancer risk more easily when determining treatment methods.

### DISCLOSURE

### Technical Problem

Although various diagnostic technologies for prostate cancer have been developed, there is a significant issue in that the accuracy of prognosis prediction is markedly lower compared to other cancer types, and there are currently no diagnostic technologies capable of accurately predicting the likelihood of metastasis or recurrence.

The objective of this invention is to provide a method for delivering improved information on cancer risk compared to existing diagnostic methods by applying a combination marker-comprising predictive genes and signaling pathway scores-to a statistical algorithm, thereby enabling the early prediction of the likelihood of metastasis or recurrence and the rapid determination of treatment methods.

### Technical Solution

Hereinafter, various embodiments described herein are described with reference to the drawings. In the following description, various specific details, such as specific forms, compositions, and processes, are provided for a complete understanding of the present invention. However, a particular embodiment may be practiced without one or more of these specific details, or in combination with other known methods and forms. In other instances, well-known processes and manufacturing techniques are not described in specific detail to avoid unnecessarily obscuring the invention. References throughout this specification to "one embodiment" or "embodiments" mean that the particular features, forms, compositions, or characteristics described in connection with the embodiment are included in one or more embodiments of the invention. Therefore, the context of "in one embodiment" or "embodiment" as expressed at various locations throughout this specification does not necessarily refer to the same embodiment of the invention. Furthermore, specific features, forms, compositions, or characteristics may be combined in any suitable manner in one or more embodiments.

Unless otherwise defined herein, all scientific and technical terms used in this specification have the same meaning as is commonly understood by those skilled in the art in the technical field to which the present invention pertains.

In this specification, "Next Generation Sequencing (NGS)" refers to a method of analyzing the entire nucleotide sequence by dividing the genome into countless fragments, decoding the genetic information of each fragment, and reassembling them. It has the advantage of enabling high-speed analysis of the genome's nucleotide sequence and is also referred to as High-throughput sequencing, Massive parallel sequencing, or Second-generation sequencing. Compared to NGS, Sanger sequencing can also read the entire human genome, but since it can only target known genes, it is limited in scope, and repeated experiments are required to examine multiple genes. For example, compared to Sanger sequencing, which must be performed in approximately 3 million separate runs, NGS is a significantly improved analytical method in terms of both time and cost. Mass parallel sequencing, made possible by next-generation sequencing (NGS) technology, is another method for quantifying RNA transcripts in tissue samples, and RNA-sequencing is a technique that utilizes this approach. It is currently the most powerful analytical tool used in transcriptomics, including for detecting differences in gene expression levels between different physiological conditions or changes occurring during development or disease progression. Specifically, RNA-sequencing can be used to study phenomena such as changes in gene expression, alternative splicing events, allele-specific gene expression and gene fusion phenomena, as well as chimeric transcripts, including novel transcripts and RNA editing.

In this specification, the term "prognosis" refers to a broad concept encompassing the course of the disease-including tumor migration and invasion within tissues, metastasis to other tissues, recurrence, and disease-related mortality-as well as the likelihood of cure. For the purposes of the present invention, prognosis refers to the disease course or survival prognosis of patients with solid tumors, specifically prostate cancer. Using the method of the present invention, the survival prognosis for prostate cancer can be easily assessed, allowing for an easy decision regarding the use of additional treatment methods. Ultimately, this can improve survival rates following the onset of prostate cancer. More specifically, the prognosis sought to be predicted in the present invention includes the likelihood of recurrence, metastasis, or a combination thereof following surgical resection.

In this specification, the term "metastasis" refers to the process by which cancer cells detach from the primary tumor (the tumor where the cancer first originated) and migrate to other parts of the body, forming new tumors at those sites. Metastasis represents a critical stage in which the cancer becomes more lethal; it is known that more than 90% of cancer-related deaths are caused by metastasis. The present invention enables a more precise diagnosis of cancer metastasis, particularly in the case of prostate cancer.

In this specification, the term "recurrence" refers to the phenomenon where cancer, which appeared to have been cured for a certain period after treatment, reappears. Since this occurs when cancer cells remaining after treatment grow again over time to form new tumors, early diagnosis is crucial. Depending on the location where the cancer recurs, it is classified into local recurrence, regional recurrence, and distant recurrence. Specifically, recurrence in the present invention may be biochemical recurrence (BCR), but is not limited thereto.

In this specification, the term "biochemical recurrence" generally refers to a state in which a signal indicating that the cancer has become active again after prostate cancer treatment is detected through biochemical markers. Following radical prostatectomy, biochemical recurrence is considered to have occurred if PSA levels are measured at 0.2 ng/mL or higher on two or more consecutive occasions; following radiation therapy, biochemical recurrence is defined as a rise in PSA of 2 ng/mL or more above the post-treatment nadir. Although BCR may serve as an early warning sign of cancer recurrence, it does not necessarily correspond to clinically apparent recurrence (e.g., symptoms, lesions on imaging). When BCR is detected, treatment decisions are made by comprehensively considering additional tests and the patient's condition.

In this specification, the term "prediction" refers to the act of forecasting the course and outcome of a disease in advance. More specifically, prognosis prediction can be interpreted as any act of predicting the course of the disease after treatment by comprehensively considering the patient's physiological or environmental status, as the course of the disease after treatment may vary depending on such factors.

According to one embodiment of the present invention, the invention relates to a composition for predicting the prognosis of cancer and a composition for predicting the likelihood of cancer metastasis or recurrence.

In the present invention, the composition may comprise an agent that measures the expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or the protein encoded thereby.

In the present invention, the composition may further comprise an agent that additionally measures the expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or the protein encoded thereby.

Among the aforementioned genes of the present invention, ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2 are characterized as genes related to signaling pathways.

In the present invention, the signaling pathway may be associated with cancer cell death, inhibition of cancer cell proliferation, metastasis, or recurrence, specifically in prostate cancer, but is not limited thereto.

In the present invention, the term "signaling pathway-related gene" refers to a gene involved in the aforementioned cancer-related signaling pathways or a gene from which information regarding activation or inactivation of the corresponding pathways can be obtained based on its expression patterns.

In the present invention, the signaling pathway may be at least one selected from the group consisting of the adipogenesis pathway, the apoptosis pathway II, the E2F targets pathway, the estrogen response early pathway, the estrogen response late pathway, the NOTCH signaling pathway, the WNT beta catenin signaling pathway, the MAPK signaling pathway, the ErbB signaling pathway, the Ras signaling pathway, the Rap1 signaling pathway, the chemokine signaling pathway, the NF-kappa B signaling pathway, the cell cycle pathway, the p53 signaling pathway, the mTOR signaling pathway, the PI3K-Akt signaling pathway, the apoptosis pathway I, the ferroptosis pathway, the necroptosis pathway, the cellular senescence pathway, the Wnt signaling pathway, the Notch signaling pathway, the TGF-beta signaling pathway, the JAK-STAT signaling pathway, the T cell receptor signaling pathway, the B cell receptor signaling pathway, the TNF signaling pathway, the transcriptional misregulation pathway in cancer, the prostate cancer pathway, the ESR-mediated signaling pathway, the estrogen-dependent gene expression pathway, and the androgen receptor network pathway in prostate cancer, but is not limited thereto.

In the composition of the present invention, the composition may comprise an agent for measuring the expression level of a gene, wherein the agent includes one or more members selected from the group consisting of primers, probes, and antisense nucleotides that specifically bind to the gene, but is not limited thereto.

In the composition of the present invention, the composition may comprise an agent for measuring the expression level of a protein encoded by the gene, wherein the agent includes one or more members selected from the group consisting of antibodies, oligopeptides, ligands, peptide nucleic acids (PNA), and aptamers that specifically bind to the protein, but is not limited thereto.

In the present invention, the cancer may be at least one selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and may specifically be prostate cancer, but is not limited thereto.

According to another embodiment of the present invention, the present invention relates to a kit for predicting cancer prognosis or a kit for predicting the likelihood of metastasis or recurrence of cancer, comprising the composition.

In the present invention, the kit may be, but is not limited to, an NGS kit, an RT-PCR kit, a DNA chip kit, an RNA sequencing kit, an ELISA kit, a protein chip kit, or a rapid kit.

The kit of the present invention may further comprise one or more additional components, compositions, solutions, or devices suitable for the analytical method.

For example, in the present invention, the kit may further comprise essential components necessary for performing a reverse transcription polymerase chain reaction. The reverse transcription polymerase chain reaction kit includes a primer pair specific to a gene encoding a marker protein. The primers are nucleotides having sequences specific to the nucleic acid sequence of the gene and may have a length of about 7 bp to 50 bp, more preferably about 10 bp to 30 bp. The kit may also include primers specific to the nucleic acid sequence of a control gene. In addition, the reverse transcription polymerase chain reaction kit may include test tubes or other suitable containers, reaction buffers (pH and magnesium concentration may vary), deoxynucleotides (dNTPs), enzymes such as Taq polymerase and reverse transcriptase, DNase, RNase inhibitors, DEPC-treated water, and sterile water.

Furthermore, the kit of the present invention may include essential components required for performing a DNA chip assay. A DNA chip kit may include a substrate to which cDNA or oligonucleotides corresponding to a gene or a fragment thereof are attached, as well as reagents, formulations, enzymes, and the like for producing fluorescently labeled probes. Furthermore, the substrate may include cDNA or oligonucleotides corresponding to a control gene or a fragment thereof.

Furthermore, the kit of the present invention may include essential components necessary to perform an ELISA. The ELISA kit may include an antibody specific to the protein. The antibody may be a monoclonal antibody, polyclonal antibody, or recombinant antibody exhibiting high specificity and affinity for the marker protein and minimal cross-reactivity with other proteins. Furthermore, the ELISA kit may include an antibody specific to a control protein. In addition, the ELISA kit may include reagents capable of detecting bound antibodies, such as labeled secondary antibodies, chromophores, enzymes (e.g., conjugated to antibodies), and their substrates, or other substances capable of binding to antibodies.

In the kit of the present invention, immobilized supports for antigen-antibody binding may include, but are not limited to, nitrocellulose membranes, PVDF membranes, well plates made of polyvinyl resin or polystyrene resin, and glass slides.

Furthermore, in the kit of the present invention, labels for secondary antibodies may include conventional chromogenic or detectable labels, including HRP (horseradish peroxidase), alkaline phosphatase, colloidal gold, FITC (poly-L-lysine-fluorescein isothiocyanate), RITC (rhodamine B isothiocyanate), and other fluorescent substances and dyes, but are not limited thereto.

Furthermore, the chromogenic substrate used to induce color development in the kit of the present invention may be selected depending on the specific label and may include TMB (3,3',5,5'-tetramethylbenzidine), ABTS [2,2'-azino-bis(3-ethylbenzothiazoline-6-sulfonic acid)], and OPD (o-phenylenediamine). Preferably, the chromogenic substrate is provided dissolved in a buffer solution (0.1 M NaAc, pH 5.5). Chromogenic substrates such as TMB are oxidized by HRP, which is used as a label on the secondary antibody conjugate, to produce a colored precipitate, and the presence or absence of the marker proteins can be detected by visually confirming the degree of precipitation.

In the kit of the present invention, the washing solution preferably comprises a phosphate buffer, NaCl, and Tween 20, and more preferably comprises a buffer solution (PBST) consisting of 0.02 M phosphate buffer, 0.13 M NaCl, and 0.05% Tween 20. The washing solution is used to wash the antigen-antibody complex 3 to 6 times after the antigen-antibody binding reaction and subsequent reaction with a secondary antibody, by adding an appropriate amount to the immobilized support. A sulfuric acid solution (H₂SO₄) may be used as a reaction stopping solution.

According to another embodiment of the present invention, the present invention relates to a method for providing information on cancer prognosis and a method for providing information on the likelihood of metastasis or recurrence of cancer.

In the present invention, the method may comprise measuring, in a biological sample obtained from a subject, the expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFAT1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

In the present invention, the method may further comprise measuring, in a biological sample obtained from a subject, the expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.

According to the present invention, measurement of the expression level may be performed by confirming the presence and degree of expression of mRNA from the gene group, and may be carried out by measuring the expression level of the relevant gene from mRNA extracted from a sample obtained from the subject. Analytical methods for measuring the expression level may include RT-PCR, competitive RT-PCR, real-time RT-PCR, RNase protection assay (RPA), northern blotting, and DNA microarray analysis, but are not limited thereto, and any suitable method commonly used in the art may be employed.

Agents for measuring the expression level of mRNA according to the present invention are preferably antisense oligonucleotides, primers, or probes, and primers or probes capable of specifically amplifying a specific region of the genes may be designed based on the nucleotide sequences of the gene group. Since the nucleotide sequences of the gene group according to the present invention are registered in GenBank and are known in the art, a person skilled in the art can design antisense oligonucleotides, primers, or probes capable of specifically amplifying a specific region of the genes based on the nucleotide sequences.

In the method for calculating the expression levels of differentially expressed genes according to the present invention, the expression level of each gene or transcript in each sample may be determined using the number of reads mapped by RNA sequencing. However, defining the expression level based solely on the number of mapped reads may introduce sample-specific errors and may not provide an objective value. Therefore, more preferably, a normalization process may be performed in order to derive an objective value.

In the present invention, the cancer may be at least one selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and may specifically be prostate cancer.

The method for providing information on cancer prognosis according to the present invention may be used not only to predict the cancer prognosis of a subject but also to predict the likelihood of cancer metastasis and, further, the likelihood of cancer recurrence in the subject.

According to another embodiment of the present invention, there is provided a cancer prognosis diagnostic device comprising: (a) a detection unit configured to measure and normalize the expression levels of a gene group or a group of proteins encoded thereby in a biological sample obtained from a subject; and (b) an output unit configured to predict and output cancer prognosis.

In the present invention, the detection unit may measure, from the biological sample obtained from the subject, the expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

In the present invention, the detection unit may further measure, from the biological sample obtained from the subject, the expression level of a signaling pathway-related gene or a protein encoded thereby.

In the present invention, the signaling pathway may be associated with cancer cell death, inhibition of cancer cell proliferation, metastasis, or recurrence, specifically in prostate cancer, but is not limited thereto.

In the present invention, the term "signaling pathway-related gene" refers to a gene involved in the aforementioned cancer-related signaling pathways or a gene from which information regarding activation or inactivation of the corresponding pathway can be obtained based on its expression pattern.

In the present invention, the signaling pathway may be at least one selected from the group consisting of the adipogenesis pathway, the apoptosis pathway II, the E2F targets pathway, the estrogen response early pathway, the estrogen response late pathway, the NOTCH signaling pathway, the WNT beta catenin signaling pathway, the MAPK signaling pathway, the ErbB signaling pathway, the Ras signaling pathway, the Rap 1 signaling pathway, the chemokine signaling pathway, the NF-kappa B signaling pathway, the cell cycle pathway, the p53 signaling pathway, the mTOR signaling pathway, the PI3K-Akt signaling pathway, the apoptosis pathway I, the ferroptosis pathway, the necroptosis pathway, the cellular senescence pathway, the Wnt signaling pathway, the Notch signaling pathway, the TGF-beta signaling pathway, the JAK-STAT signaling pathway, the T cell receptor signaling pathway, the B cell receptor signaling pathway, the TNF signaling pathway, the transcriptional misregulation pathway in cancer, the prostate cancer pathway, the ESR-mediated signaling pathway, the estrogen-dependent gene expression pathway, and the androgen receptor network pathway in prostate cancer, but is not limited thereto.

In the present invention, the cancer may be at least one selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and may specifically be prostate cancer.

According to another embodiment of the present invention, the present invention relates to a method for providing information regarding cancer risk.

In the present invention, the method may be based on a Cox regression analysis algorithm.

In the present invention, the method may include calculating gene expression levels and signaling pathway scores for a biological sample obtained from a subject and classifying them as explanatory variables.

In the present invention, the term "Cox regression analysis" refers to one of the survival analysis methods first developed by Cox in 1972 and is a statistical technique that serves as an important tool in survival analysis by analyzing the relationship between survival time and one or more predictor variables. It enables analysis of the risk of an event occurring over time in relation to various factors. In particular, it is widely used in the medical field to evaluate patient survival rates and treatment effects, and is one of the useful tools for handling data that satisfy the proportional hazards assumption.

In the present invention, the term "cancer" generally refers to a physiological condition in mammals characterized by uncontrolled cell proliferation. Examples of cancer include prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and may specifically be prostate cancer, but is not limited thereto.

In the present invention, the term "cancer risk" may broadly refer to the probability that a specific individual will develop cancer, the probability that a specific individual will experience metastasis or recurrence of cancer, or the likelihood that a specific individual will not respond favorably to anticancer treatment, based solely on genetic risk assessment excluding environmental and lifestyle factors, and may specifically refer to the probabilistic likelihood of cancer metastasis or recurrence, but is not limited thereto.

In the present invention, the term "subject" refers to an individual in whom a disease has developed or is likely to develop, and may be a mammal including a human. For example, the subject may be selected from the group consisting of a human, rat, mouse, guinea pig, hamster, rabbit, monkey, dog, cat, cow, horse, pig, sheep, and goat, and may specifically be a human, but is not limited thereto.

In the present invention, the term "biological sample" refers to any sample from which a subject's genetic information can be identified, and may include, but is not limited to, a solid tissue sample, tissue culture supernatant, liquid tissue sample, cell, or cell fragment. Further, non-limiting examples of the biological sample may include at least one selected from the group consisting of whole blood, leukocytes, peripheral blood mononuclear cells, buffy coat, plasma, serum, sputum, tears, mucus, nasal washes, nasal aspirates, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, and cerebrospinal fluid, but is not limited thereto as long as nucleic acid can be extracted therefrom.

In the present invention, the term "gene expression level" refers to the amount of DNA gene sequence information converted into transcribed RNA (an initial unspliced RNA transcript or mature mRNA) or an encoded protein product. The gene expression level may be monitored by measuring total RNA or a protein product of the gene, or a subsequent level thereof.

In the present invention, the term "signaling pathway" refers to a signaling pathway related to cancer cell death, inhibition of cancer cell proliferation, metastasis, or recurrence, and specifically may refer to a signaling pathway related to prostate cancer cell death, inhibition of proliferation, metastasis, or recurrence, but is not limited thereto.

In the present invention, the signaling pathway may be at least one selected from the group consisting of the adipogenesis pathway, the apoptosis pathway II, the E2F targets pathway, the estrogen response early pathway, the estrogen response late pathway, the NOTCH signaling pathway, the WNT beta catenin signaling pathway, the MAPK signaling pathway, the ErbB signaling pathway, the Ras signaling pathway, the Rap1 signaling pathway, the chemokine signaling pathway, the NF-kappa B signaling pathway, the cell cycle pathway, the p53 signaling pathway, the mTOR signaling pathway, the PI3K-Akt signaling pathway, the apoptosis pathway I, the ferroptosis pathway, the necroptosis pathway, the cellular senescence pathway, the Wnt signaling pathway, the Notch signaling pathway, the TGF-beta signaling pathway, the JAK-STAT signaling pathway, the T cell receptor signaling pathway, the B cell receptor signaling pathway, the TNF signaling pathway, the transcriptional misregulation pathway in cancer, the prostate cancer pathway, the ESR-mediated signaling pathway, the estrogen-dependent gene expression pathway, and the androgen receptor network pathway in prostate cancer, but is not limited thereto.

In the present invention, the term "explanatory variable" refers to a concept as defined herein and denotes the expression level of differentially expressed genes (DEGs) or a signaling pathway score. Examples thereof can be readily understood with reference to the explanatory variable items listed in Tables 4 and 5 of the Examples of this specification.

In the present invention, the gene expression level refers to the expression level of differentially expressed genes, and the expression level may be calculated using the number of reads mapped by RNA sequencing to determine the expression level of each gene or transcript in each sample. However, defining the expression level based solely on the number of mapped reads may introduce sample-specific errors and may not provide an objective value. Therefore, more preferably, the expression level may be derived by performing a normalization process in order to obtain an objective value.

In the present invention, the signaling pathway score may be calculated using GSVA (Gene Set Variation Analysis) based on the signaling pathway-related genes listed in Table 2 that are included in each signaling pathway.

In the present invention, the method may further comprise calculating a risk score according to Equation 1 using a Cox regression analysis algorithm based on a combination of at least one gene selected from Table 1 of this specification and at least one signaling pathway selected from Table 2 of this specification.
$Risk Score = {\sum }_{i = 1}^{p} Regression {Coefficients}_{i} \times Explanatory {Variables}_{i}$

In Equation 1,
p represents the number of explanatory variables, and each explanatory variable represents a gene expression level or a signaling pathway score.

In the present invention, the regression coefficients in Equation 1 may include values within ±5% of the respective regression coefficient values corresponding to each explanatory variable in Table 4 or Table 5 of this specification. More specifically, the values may fall within ±4%, ±3%, ±2%, or ±1% of the respective regression coefficient values.

As a non-limiting example, the regression coefficient value corresponding to the EEF1A1 gene in Table 4 of this specification is -24.5157, and applying the margin of error to this value, the regression coefficient may include values ranging from -25.7415 to -23.2899.

As another non-limiting example, the regression coefficient value corresponding to the p53 signaling pathway (hsa04115_p53_signaling_pathway) in Table 5 of this specification is - 12.2718, and applying the margin of error to this value, the regression coefficient may include values ranging from -12.8854 to -11.6582.

In the present invention, the aforementioned genes may be DEG genes according to an embodiment of the present invention, specifically ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414.

In the present invention, the signaling pathway-related genes refer to genes that are involved in the aforementioned cancer-related signaling pathways or from which information regarding the activation or inactivation of said pathways can be obtained through their expression patterns. Specifically, ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2.

The published Gene ID information (ensembl_gene_id) for the signaling pathway-related genes of the present invention is as follows:
ACTB (ensembl_gene_id: ENSG00000075624), H3-3B (ensembl_gene_id: ENSG00000132475), ACLY (ensembl_gene_id: ENSG00000131473), ACOX1 (ensembl_gene_id: ENSG00000161533), ACSL4 (ensembl_gene_id: ENSG00000068366), ACVR1 (ensembl_gene_id: ENSG00000115170), AKT1 (ensembl_gene _id: ENSG00000142208), AXIN1 (ensembl_gene_id: ENSG00000103126), AXIN2 (ensembl_gene_id: ENSG00000168646), BCL2L1 (ensembl_gene_id: ENSG00000171552), BMP4 (ensembl_gene_id: ENSG00000125378), BMPR1A (ensembl_gene_id: ENSG00000107779), BRCA1 (ensembl_gene_id: ENSG00000012048), BTK (ensembl_gene_id: ENSG00000010671), CASP3 (ensembl_gene_id: ENSG00000164305), CASP8 (ensembl_gene_id: ENSG00000064012), CASP9 (ensembl_gene_id: ENSG00000132906), CCNA2 (ensembl_gene_id: ENSG00000145386), CCNB2 (ensembl_gene_id: ENSG00000157456), CCND1 (ensembl_gene_id: ENSG00000110092), CCR5 (ensembl_gene_id: ENSG00000160791), CD40 (ensembl_gene_id: ENSG00000101017), CDK1 (ensembl_gene_id: ENSG00000170312), CDK2 (ensembl_gene_id: ENSG00000123374), CDK4 (ensembl_gene_id: ENSG00000135446), CDKN1A (ensembl_gene_id: ENSG00000124762), CDKN2A (ensembl_gene_id: ENSG00000147889), CHEK1 (ensembl_gene_id: ENSG00000149554), CS (ensembl_gene_id: ENSG00000062485), CTNNB1 (ensembl_gene_id: ENSG00000168036), CXCR4 (ensembl_gene_id: ENSG00000121966), DVL1 (ensembl_gene_id: ENSG00000107404), DVL2 (ensembl_gene_id: ENSG00000004975), EP300 (ensembl_gene_id: ENSG00000100393), FYN (ensembl_gene_id: ENSG00000010810), GADD45A (ensembl_gene_id: ENSG00000116717), GNB1 (ensembl_gene_id: ENSG00000078369), GPX4 (ensembl_gene_id: ENSG00000167468), GRB2 (ensembl_gene_id: ENSG00000177885), GSK3B (ensembl_gene_id: ENSG00000082701), H3C12 (ensembl_gene_id: ENSG00000197153), H3C13 (ensembl_gene_id: ENSG00000183598), H4C6 (ensembl_gene_id: ENSG00000274618), HMOX1 (ensembl_gene_id: ENSG00000100292), HRAS (ensembl_gene_id: ENSG00000174775), IKBKB (ensembl_gene_id: ENSG00000104365), IKBKG (ensembl_gene_id: ENSG00000269335), IL1B (ensembl_gene_id: ENSG00000125538), IL6 (ensembl_gene_id: ENSG00000136244), INS (ensembl_gene_id: ENSG00000254647), JAG1 (ensembl_gene_id: ENSG00000101384), JAK1 (ensembl_gene_id: ENSG00000162434), JAK2 (ensembl_gene_id: ENSG00000096968), JAK3 (ensembl_gene_id: ENSG00000105639), JUN (ensembl_gene_id: ENSG00000177606), KRAS (ensembl_gene_id: ENSG00000133703), LCK (ensembl_gene_id: ENSG00000182866), LEF1 (ensembl_gene_id: ENSG00000138795), LPCAT3 (ensembl_gene_id: ENSG00000111684), LYN (ensembl_gene_id: ENSG00000254087), MAML1 (ensembl_gene_id: ENSG00000161021), MAML2 (ensembl_gene_id: ENSG00000184384), MAML3 (ensembl_gene_id: ENSG00000196782), MAPK1 (ensembl_gene_id: ENSG00000100030), MAPK3 (ensembl_gene_id: ENSG00000102882), MCM7 (ensembl_gene_id: ENSG00000166508), MLST8 (ensembl_gene_id: ENSG00000167965), MTOR (ensembl_gene_id: ENSG00000198793), NCOA4 (ensembl_gene_id: ENSG00000266412), NFKB1 (ensembl_gene_id: ENSG00000109320), NFKBIA (ensembl_gene_id: ENSG00000100906), NOTCH1 (ensembl_gene_id: ENSG00000148400), NOTCH2 (ensembl_gene_id: ENSG00000134250), NOTCH3 (ensembl_gene_id: ENSG00000074181), NOTCH4 (ensembl_gene_id: ENSG00000204301), NRAS (ensembl_gene_id: ENSG00000213281), PIK3CA (ensembl_gene_id: ENSG00000121879), PIK3R1 (ensembl_gene_id: ENSG00000145675), PLCG2 (ensembl_gene_id: ENSG00000197943), PTEN (ensembl_gene_id: ENSG00000171862), RBPJ (ensembl_gene_id: ENSG00000168214), RELA (ensembl_gene_id: ENSG00000173039), RHEB (ensembl_gene_id: ENSG00000106615), RHOA (ensembl_gene_id: ENSG00000067560), RPTOR (ensembl_gene_id: ENSG00000141564), RUNX1 (ensembl_gene_id: ENSG00000159216), SDHB (ensembl_gene_id: ENSG00000117118), SMAD2 (ensembl_gene_id: ENSG00000175387), SMAD3 (ensembl_gene_id: ENSG00000166949), SMAD4 (ensembl_gene_id: ENSG00000141646), SOS1 (ensembl_gene_id: ENSG00000115904), SRC (ensembl_gene_id: ENSG00000197122), STAT1 (ensembl_gene_id: ENSG00000115415), STAT3 (ensembl_gene_id: ENSG00000168610), SYK (ensembl_gene_id: ENSG00000165025), TFRC (ensembl_gene_id: ENSG00000072274), TGFB1 (ensembl_gene_id: ENSG00000105329), TNF (ensembl_gene_id: ENSG00000232810), TNFRSF1A (ensembl_gene_id: ENSG00000067182), TP53 (ensembl_gene_id: ENSG00000141510), TRAF6 (ensembl_gene_id: ENSG00000175104), TSC2 (ensembl_gene_id: ENSG00000103197), and TYK2 (ensembl_gene_id: ENSG00000105397)

In the present invention, the genes related to the adipogenesis pathway among the aforementioned signaling pathways may include, but are not limited to, at least one selected from the group consisting of ACLY, ACOX1, CS, GADD45A, GPX4, LPCAT3, and SDHB.

In the present invention, the gene related to the apoptosis pathway II among the signaling pathways may include at least one selected from the group consisting of BCL2L1, BRCA1, CASP3, CASP8, CASP9, CCND1, CDK2, CDKN1A, CTNNB1, GADD45A, GPX4, HMOX1, IL1B, IL6, JUN, LEF1, RELA, and TNF, but is not limited thereto.

In the present invention, the genes related to the E2F target pathway among the aforementioned signaling pathways may include, but are not limited to, at least one selected from the group consisting of BRCA1, CCNB2, CDK1, CDK4, CDKN1A, CDKN2A, CHEK1, MCM7, TFRC, and TP53.

In the present invention, the gene related to the Estrogen Response Early pathway among the signaling pathways may include at least one selected from the group consisting of CCND1 and JAK2, but is not limited thereto.

In the present invention, the genes related to the Estrogen Response Late pathway among the aforementioned signaling pathways may include at least one selected from the group consisting of CCND1, JAK1, and JAK2, but are not limited thereto.

In the present invention, the gene related to the NOTCH signaling pathway among the aforementioned signaling pathways may include at least one selected from the group consisting of CCND1, DVL1, DVL2, EP300, JAG1, MAML1, MAML2, MAML3, NOTCH1, NOTCH2, NOTCH3, NOTCH4, and RBPJ, but is not limited thereto.

In the present invention, the genes related to the WNT beta catenin signaling pathway among the aforementioned signaling pathways may include at least one selected from the group consisting of AXIN1, AXIN2, CTNNB1, DVL2, JAG1, LEF1, MAML1, NOTCH1, NOTCH4, RBPJ, and TP53, but is not limited thereto.

In the present invention, the genes related to the MAPK signaling pathway among the aforementioned signaling pathways may include at least one selected from the group consisting of AKT1, CASP3, GADD45A, GRB2, HRAS, IKBKB, IKBKG, IL1B, INS, JUN, KRAS, MAPK1, MAPK3, NFKB1, NRAS, RELA, SOS1, TGFB1, TNF, TNFRSF1A, TP53, and TRAF6, but is not limited thereto.

In the present invention, the ErbB signaling pathway-related genes among the aforementioned signaling pathways may include, but are not limited to, at least one selected from the group consisting of AKT1, CDKN1A, GRB2, GSK3B, HRAS, JUN, KRAS, MAPK1, MAPK3, MTOR, NRAS, PIK3CA, PIK3R1, PLCG2, SOS1, and SRC, but is not limited thereto.

In the present invention, the genes related to the Ras signaling pathway among the aforementioned signaling pathways may include at least one selected from the group consisting of AKT1, BCL2L1, GNB1, GRB2, HRAS, IKBKB, IKBKG, INS, KRAS, MAPK1, MAPK3, NFKB1, NRAS, PIK3CA, PIK3R1, PLCG2, RELA, RHOA, and SOS1, but is not limited thereto.

In the present invention, the genes related to the Rap1 signaling pathway among the aforementioned signaling pathways may include at least one selected from the group consisting of ACTB, AKT1, CTNNB1, HRAS, INS, KRAS, MAPK1, MAPK3, NRAS, PIK3CA, PIK3R1, RHOA, and SRC, but are not limited thereto.

In the present invention, the genes related to the chemokine signaling pathway among the aforementioned signaling pathways may include at least one selected from the group consisting of AKT1, CCR5, CXCR4, GNB1, GRB2, GSK3B, HRAS, IKBKB, IKBKG, JAK2, JAK3, KRAS, LYN, MAPK1, MAPK3, NFKB1, NFKBIA, NRAS, PIK3CA, PIK3R1, PLCG2, RELA, RHOA, SOS1, SRC, STAT1, and STAT3, but is not limited thereto.

In the present invention, the genes related to the NF-kappa B signaling pathway among the aforementioned signaling pathways include BCL2L1, BTK, CD40, GADD45A, IKBKB, IKBKG, IL1B, LCK, LYN, NFKB1, NFKBIA, PLCG2, RELA, SYK, TNF, TNFRSF1A, and TRAF6, but is not limited thereto.

In the present invention, the cell cycle pathway-related genes among the aforementioned signaling pathways may include, but are not limited to, at least one selected from the group consisting of CCNA2, CCNB2, CCND1, CDK1, CDK2, CDK4, CDKN1A, CDKN2A, CHEK1, EP300, GADD45A, GSK3B, MCM7, SMAD2, SMAD3, SMAD4, TGFB1, and TP53, but is not limited thereto.

In the present invention, the genes related to the p53 signaling pathway among the aforementioned signaling pathways may include, but are not limited to, BCL2L1, CASP3, CASP8, CASP9, CCNB2, CCND1, CDK1, CDK2, CDK4, CDKN1A, CDKN2A, CHEK1, GADD45A, PTEN, TP53, and TSC2, but is not limited thereto.

In the present invention, the genes related to the mTOR signaling pathway among the aforementioned signaling pathways may include at least one selected from the group consisting of AKT1, DVL1, DVL2, GRB2, GSK3B, HRAS, IKBKB, INS, KRAS, MAPK1, MAPK3, MLST8, MTOR, NRAS, PIK3CA, PIK3R1, PTEN, RHEB, RHOA, RPTOR, SOS1, TNF, TNFRSF1A, and TSC2, but is not limited thereto.

In the present invention, the genes related to the PI3K-Akt signaling pathway among the aforementioned signaling pathways include AKT1, BCL2L1, BRCA1, CASP9, CCND1, CDK2, CDK4, CDKN1A, GNB1, GRB2, GSK3B, HRAS, IKBKB, IKBKG, IL6, INS, JAK1, JAK2, JAK3, KRAS, MAPK1, MAPK3, MLST8, MTOR, NFKB1, NRAS, PIK3CA, PIK3R1, PTEN, RELA, RHEB, RPTOR, SOS1, SYK, TP53, and TSC2, but is not limited thereto.

In the present invention, the genes related to Apoptosis pathway I among the aforementioned signaling pathways include ACTB, AKT1, BCL2L1, CASP3, CASP8, CASP9, GADD45A, HRAS, IKBKB, IKBKG, JUN, KRAS, MAPK1, MAPK3, NFKB1, NFKBIA, NRAS, PIK3CA, PIK3R1, RELA, TNF, TNFRSF1A, and TP53, but is not limited thereto.

In the present invention, the genes related to the ferroptosis pathway among the aforementioned signaling pathways may include, but are not limited to, at least one selected from the group consisting ofACSL4, GPX4, HMOX1, LPCAT3, NCOA4, TFRC, and TP53.

In the present invention, the genes related to the necroptosis pathway among the signaling pathways may include at least one selected from the group consisting of CASP8, IL1B, JAK1, JAK2, JAK3, STAT1, STAT3, TNF, TNFRSF1A, and TYK23, but are not limited thereto.

In the present invention, the genes related to the cellular senescence pathway among the signaling pathways may include at least one selected from the group consisting of AKT1, CCNA2, CCNB2, CCND1, CDK1, CDK2, CDK4, CDKN1A, CDKN2A, CHEK1, GADD45A, HRAS, IL6, KRAS, MAPK1, MAPK3, MTOR, NFKB1, NRAS, PIK3CA, PIK3R1, PTEN, RELA, RHEB, SMAD2, SMAD3, TGFB1, TP53, and TSC2, but is not limited thereto.

In the present invention, the genes related to the Wnt signaling pathway among the aforementioned signaling pathways may include, but are not limited to, at least one selected from the group consisting of AXIN1, AXIN2, CCND1, CTNNB1, DVL1, DVL2, EP300, GSK3B, JUN, LEF1, RHOA, SMAD3, SMAD4, and TP53, but is not limited thereto.

In the present invention, the genes related to the Notch signaling pathway among the aforementioned signaling pathways may include at least one selected from the group consisting of DVL1, DVL2, EP300, JAG1, MAML1, MAML2, MAML3, NOTCH1, NOTCH2, NOTCH3, NOTCH4, and RBPJ, but are not limited thereto.

In the present invention, the gene related to the TGF-beta signaling pathway among the aforementioned signaling pathways may include at least one selected from the group consisting of ACVR1, BMP4, BMPR1A, EP300, MAPK1, MAPK3, RHOA, SMAD2, SMAD3, SMAD4, TFRC, TGFB1, and TNF, but is not limited thereto.

In the present invention, the genes related to the JAK-STAT signaling pathway among the aforementioned signaling pathways may include at least one selected from the group consisting of AKT1, BCL2L1, CCND1, CDKN1A, EP300, GRB2, HRAS, IL6, JAK1, JAK2, JAK3, MTOR, PIK3CA, PIK3R1, SOS1, STAT1, STAT3, and TYK2, but is not limited thereto.

In the present invention, the genes related to the T cell receptor signaling pathway among the aforementioned signaling pathways may include at least one selected from the group consisting of AKT1, CDK4, FYN, GRB2, GSK3B, HRAS, IKBKB, IKBKG, JUN, KRAS, LCK, MAPK1, MAPK3, NFKB1, NFKBIA, NRAS, PIK3CA, PIK3R1, RELA, RHOA, SOS1, and TNF, but is not limited thereto.

In the present invention, the genes related to the B cell receptor signaling pathway among the aforementioned signaling pathways may include at least one selected from the group consisting of AKT1, BTK, GRB2, GSK3B, HRAS, IKBKB, IKBKG, JUN, KRAS, LYN, MAPK1, MAPK3, NFKB1, NFKBIA, NRAS, PIK3CA, PIK3R1, PLCG2, RELA, SOS1, and SYK, but is not limited thereto.

In the present invention, the genes related to the TNF signaling pathway among the aforementioned signaling pathways may include at least one selected from the group consisting of AKT1, CASP3, CASP8, IKBKB, IKBKG, IL1B, IL6, JAG1, JUN, MAPK1, MAPK3, NFKB1, NFKBIA, PIK3CA, PIK3R1, RELA, TNF, and TNFRSF1A, but is not limited thereto.

In the present invention, the genes related to the transcriptional misregulation pathway in cancer among the aforementioned signaling pathways may include at least one selected from the group consisting of BCL2L1, CCNA2, CD40, CDKN1A, GADD45A, H3-3B, H3C12, H3C13, IL6, NFKB1, RELA, RUNX1, and TP53, but is not limited thereto.

In the present invention, genes related to the prostate cancer pathway among the aforementioned signaling pathways may include at least one selected from the group consisting of AKT1, CASP9, CCND1, CDK2, CDKN1A, CTNNB1, EP300, GRB2, GSK3B, HRAS, IKBKB, IKBKG, INS, KRAS, LEF1, MAPK1, MAPK3, MTOR, NFKB1, NFKBIA, NRAS, PIK3CA, PIK3R1, PTEN, RELA, SOS1, and TP53, but is not limited thereto.

In the present invention, the genes related to the ESR-mediated signaling pathway among the aforementioned signaling pathways include at least one selected from the group consisting of AKT1, AXIN1, CCND1, EP300, GNB1, H3-3B, H3C12, H3C13, H4C6, HRAS, JUN, KRAS, MAPK1, MAPK3, NRAS, PIK3CA, PIK3R1, RUNX1, and SRC, but is not limited thereto.

In the present invention, the genes related to the estrogen-dependent gene expression pathway among the aforementioned signaling pathways may include, but are not limited to, at least one selected from the group consisting of AXIN1, CCND1, EP300, H3-3B, H3C12, H3C13, H4C6, JUN, and RUNX1.

In the present invention, the genes related to the androgen receptor network pathway in prostate cancer among the aforementioned signaling pathways may include at least one selected from the group consisting of AKT1, BRCA1, CASP3, CASP8, CASP9, CCND1, CDK1, CDK2, CDK4, CHEK1, GRB2, HRAS, JAK1, JUN, MAPK1, MAPK3, MTOR, PIK3CA, PTEN, RHEB, RPTOR, SMAD2, SMAD3, SOS1, STAT1, STAT3, TP53, and TSC2, but is not limited thereto.

In the present invention, the method may further comprise classifying the biological sample based on the Risk score value calculated as described above.

In the present invention, the classifying step may involve dividing the Risk score, obtained by multiplying the regression coefficient corresponding to each explanatory variable by the variable-specific value, into a High group and a Low group according to the cutoff criterion disclosed in the Examples of the present invention.

In the present invention, the cutoff value serving as the cutoff criterion may be a value within ±5% of -257.50477. More specifically, a value within ±4% may be applied; more specifically, a value within ±3% may be applied; still more specifically, a value within ±2% may be applied; and most specifically, a value within ±1% may be applied.

In the present invention, the variable-specific value may be a gene expression level of Table 1 or a signaling pathway score derived from the signaling pathway-related genes of Table 2.

In the present invention, the High group may be determined to be patients who are unlikely to exhibit a beneficial response to anticancer treatment and thus are likely to have a high possibility of cancer metastasis or a high risk of biochemical recurrence; specifically, the High group may be determined to be patients who are likely to have a high possibility of prostate cancer metastasis or a high risk of biochemical recurrence.

In the present invention, the Low group may be determined to be patients who are likely to exhibit a beneficial response to anticancer treatment and thus are likely to have a low possibility of cancer metastasis or a low risk of biochemical recurrence; specifically, the Low group may be determined to be patients who are likely to have a low possibility of prostate cancer metastasis or a low risk of biochemical recurrence.

In the present invention, the term "beneficial response" refers to an improvement in any measure of patient status, such as overall survival, long-term survival, recurrence-free survival, and distant recurrence-free survival, which are commonly used measures in the art. Recurrence-free survival (RFS) refers to the time, in months, from surgery to the first local recurrence, regional recurrence, or distant recurrence. Distant recurrence-free survival (DRFS) or distant metastasis-free survival (DMFS) refers to the time, in months, from surgery to the first distant recurrence. Recurrence refers to RFS and/or DRFS. In this specification, the term "long-term survival" refers to survival for at least 3 years, at least 5 years, at least 8 years, or at least 10 years after surgery or other treatment.

According to another embodiment of the present invention, the invention relates to a diagnostic device for predicting cancer risk.

In the present invention, the diagnostic device comprises: (a) an input unit configured to receive gene expression levels and signaling pathway scores for a biological sample obtained from a subject; (b) a processing unit configured to calculate a Risk score according to Equation 1 using a Cox regression analysis algorithm based on a combination of at least one gene selected from Table 1 and at least one signaling pathway selected from Table 2; and (c) an output unit configured to classify the biological sample into a High group or a Low group based on the calculated risk score value, and to output the classification result.

$Risk Score = {\sum }_{i = 1}^{p} Regression {Coefficients}_{i} \times Explanatory {Variables}_{i}$

In the present invention, the term "diagnostic device" refers to a device capable of diagnosing a disease in vitro based on substances produced by the human body, such as blood, saliva, or urine, and may include, for example, an input unit, a processing unit, and an output unit, without limitation, as long as the device is capable of analyzing gene or protein expression levels from the aforementioned substances.

In the diagnostic device of the present invention, descriptions regarding Cox regression analysis, cancer, cancer risk, a subject, a biological sample, genes, signaling pathways, signaling pathway scores, regression coefficients, explanatory variables, the High group, and the Low group are identical to those described in the method for providing information on cancer risk, and thus are omitted herein to avoid unnecessary complexity of the specification.

### Advantageous Effects

By applying a statistical algorithm to a newly identified combination of markers capable of accurately predicting cancer risk, particularly the likelihood of metastasis or recurrence of prostate cancer, information regarding cancer risk can be provided, specifically information regarding prostate cancer risk and the likelihood of m etastasis or recurrence thereof. By predicting the likelihood of metastasis or recurrence at an early stage, treatment decisions can be made promptly, thereby significantly contributing to the improvement of patient survival rates.

### Brief Description of Drawings

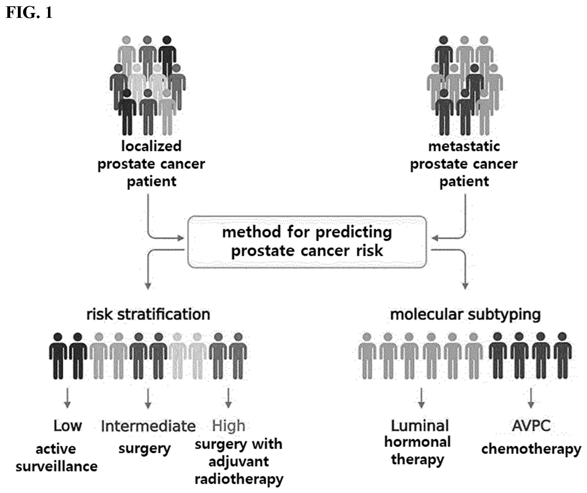

FIG. 1 is a schematic diagram of an algorithm according to an embodiment of the present invention, which first classifies the risk level of individual patients using gene expression patterns and scores of signaling pathway-related genes obtained through targeted RNA sequencing, and then determines molecular subtypes to predict drug responsiveness.
FIG. 2 shows an ROC curve illustrating the performance of a Random Forest model using only DEG markers, evaluated on a Test set, according to an embodiment of the present invention.
FIG. 3 shows an ROC curve illustrating the performance of a Random Forest model using only DEG markers, evaluated on a validation set not used in model development, according to an embodiment of the present invention.
FIG. 4 shows an ROC curve illustrating the performance of a Random Forest model using only signaling pathway-related markers, evaluated on a Test set, according to an embodiment of the present invention.
FIG. 5 shows an ROC curve illustrating the performance of a Random Forest model using only signaling pathway-related markers, evaluated on a validation set not used in model development, according to an embodiment of the present invention.
FIG. 6 shows an ROC curve illustrating the performance of a Random Forest model using a combination of DEG markers and signaling pathway-related markers, evaluated on a Test set, according to an embodiment of the present invention.
FIG. 7 shows an ROC curve illustrating the performance of a Random Forest model using a combination of DEG markers and signaling pathway-related markers, evaluated on a validation set not used in model development, according to an embodiment of the present invention.
FIG. 8a is a Kaplan-Meier plot showing the distribution of metastasis-free survival (MFS) over the entire follow-up period according to an embodiment of the present invention.
FIG. 8b is a Kaplan-Meier plot showing the distribution of biochemical recurrence-free survival (BFS) over the entire follow-up period according to an embodiment of the present invention.
FIG. 8c is a Kaplan-Meier plot showing the distribution of overall survival (OS) over the entire follow-up period according to an embodiment of the present invention.
FIG. 8d is a Kaplan-Meier plot showing the distribution of metastasis-free survival (MFS) over a 5-year follow-up period according to an embodiment of the present invention.
FIG. 8e is a Kaplan-Meier plot showing the distribution of biochemical recurrence-free survival (BFS) over a 5-year follow-up period according to an embodiment of the present invention.
FIG. 8f is a Kaplan-Meier plot showing the distribution of overall survival (OS) over a 5-year follow-up period according to an embodiment of the present invention.

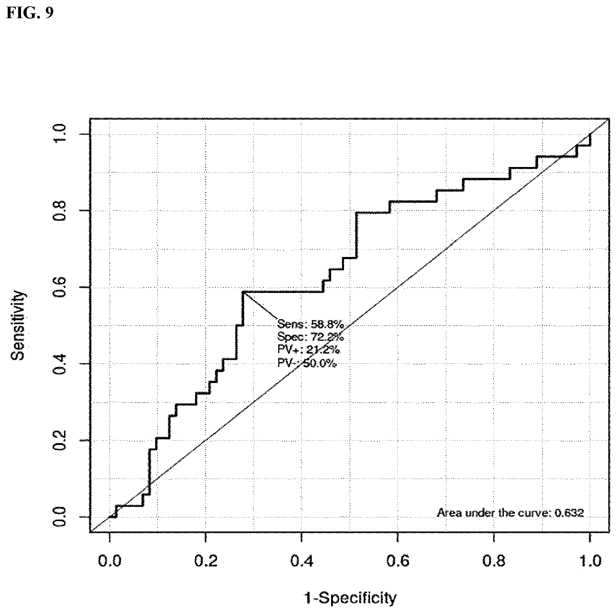

FIG. 9 shows the results of predicting the presence of metastasis based on risk scores calculated using a Cox regression analysis according to an embodiment of the present invention.

### Mode for Invention

### Examples

### Selection of Prostate Cancer Patients and Preparation of Tissue Samples

To identify genes associated with the prognosis of prostate cancer, tissue samples were obtained from patients who underwent surgery after a prostate cancer diagnosis and were classified into the following groups: patients with no evidence of disease (NED), who did not develop biochemical recurrence (BCR) or metastasis (METS); patients who experienced biochemical recurrence (BCR); and patients who developed metastasis (METS). Representative formalin-fixed paraffin-embedded (FFPE) blocks were selected from the obtained tissue samples, and RNA was extracted therefrom.

The objective was to obtain a list of genes exhibiting differences in expression levels among patients who underwent surgery after a prostate cancer diagnosis and were classified into NED, BCR, and METS groups. For this purpose, an in-house dataset of Korean prostate cancer patients was utilized. The sample data used in the in-house dataset consisted of three main patient groups: NED, BCR, and METS.

Among patients who underwent surgery for localized or locally advanced prostate cancer and were followed up, patient groups satisfying the following three conditions were selected. The first group consisted of patients (NED) who did not experience biochemical recurrence (BCR) or metastasis/recurrence for 7 years following prostatectomy. The second group consisted of patients (BCR) who experienced biochemical recurrence (defined as a consecutive increase in serum PSA to 0.2 ng/mL or higher on at least two occasions following radical prostatectomy) but did not develop metastasis during a 5-year follow-up period. The third group consisted of patients (METS) who developed metastasis during a 5-year follow-up period.

### RNA Sequencing

Prior to NGS analysis, the DQ score (DCgen Quality score, DQ score) system was used to confirm that the RNA met a predetermined quality standard for NGS library preparation based on QC information. Based on this evaluation, high-quality NGS libraries were selected to construct cDNA libraries. Gene panel probes were used to selectively detect target genes. All candidate genes were sequenced using a next-generation sequencing (NGS) platform and aligned to a public human genome reference. Reads were mapped using the STAR alignment algorithm, and gene expression levels were quantified from the aligned reads.

### Screening for markers associated with prostate cancer prognosis, metastasis, or recurrence based on RNA-sequencing expression data

To identify an optimal combination of biomarkers, a stepwise screening process was performed, including: (1) in-house data sample matching, (2) extraction of metastasis-related markers, (3) extraction of key signaling pathway-related markers associated with prostate cancer, and (4) extraction of housekeeping genes.

Patient samples used for model development were collected by matching clinical information from patients with confirmed recurrence (estimated based on tumor size, PSA levels, and Gleason score) with high clinical risk. This approach was adopted because, when samples with low clinical risk are used for model construction, clinical variables may dominate the prediction of recurrence; therefore, only gene expression levels were used as variables.

To differentiate from models based on conventional clinical information, propensity score matching (PSM) was performed to ensure similar distributions of (1) cancer stage, (2) pretreatment serum PSA levels, and (3) Gleason scores. PSM was performed using the MatchIt package in R with a nearest-neighbor matching criterion and a caliper of 0.8. Prior to matching, ANOVA and Kruskal tests confirmed the necessity of matching, as inter-institutional differences showed p-values of 0.05 or less. To enable comparison across samples, z-score normalization was applied to log2(TPM) values.

To identify genes whose expression levels were increased or decreased in patients with metastasis compared to NED patients, Limma, a DEG (Differentially Expressed Gene) analysis tool, was used. Genes satisfying both a p-value of 0.05 or less and an absolute log2(fold change) of 0.585 or greater were selected. Genes showing differential expression according to metastasis status were identified. To further validate these genes, additional genes were selected by incorporating metastasis-related clinical factors, such as ISUP grade and T-stage, followed by comparative analysis.

A total of 103 significant genes were identified, as shown in Table 1. GSEA analysis confirmed that the selected genes had high interpretability in terms of biological mechanisms and strong relevance to prostate cancer metastasis. Accordingly, these genes were selected as an optimal biomarker combination for diagnosing prostate cancer metastasis.

**[Table 1]**

| **gene_id** | **gene_name** | **logFC** | **P value** | **abs_logFC** |
|---|---|---|---|---|
| ENSG00000004776 | HSPB6 | -0.851378189 | 0.002038878 | 0.851378189 |
| ENSG00000008710 | PKD1 | -0.589536256 | 0.007958642 | 0.589536256 |
| ENSG00000034510 | TMSB10 | 2.318848415 | 5.0705E-11 | 2.318848415 |
| ENSG00000061938 | TNK2 | -0.640086605 | 3.68972E-06 | 0.640086605 |
| ENSG00000067225 | PKM | 0.707083049 | 1.53094E-05 | 0.707083049 |
| ENSG00000071127 | WDR1 | 0.59769309 | 2.06496E-07 | 0.59769309 |
| ENSG00000072310 | SREBF1 | -1.038693545 | 0.00090451 | 1.038693545 |
| ENSG00000074800 | ENO1 | 0.840850772 | 1.09025E-06 | 0.840850772 |
| ENSG00000075624 | ACTB | 3.454119503 | 4.9876E-07 | 3.454119503 |
| ENSG00000084207 | GSTP1 | 0.798458526 | 0.002504518 | 0.798458526 |
| ENSG00000087086 | FTL | 0.929758972 | 0.022950187 | 0.929758972 |
| ENSG00000089220 | PEBP1 | 1.151713824 | 3.422E-08 | 1.151713824 |
| ENSG00000090006 | LTBP4 | -0.860553947 | 0.009746852 | 0.860553947 |
| ENSG00000092199 | HNRNPC | 0.891272379 | 1.91104E-07 | 0.891272379 |
| ENSG00000092841 | MYL6 | 0.896046463 | 0.002872848 | 0.896046463 |
| ENSG00000099977 | DDT | 0.814582099 | 1.6297E-07 | 0.814582099 |
| ENSG00000101439 | CST3 | 0.730651508 | 0.000200604 | 0.730651508 |
| ENSG00000102878 | HSF4 | -0.732274588 | 4.08602E-05 | 0.732274588 |
| ENSG00000104419 | NDRG1 | 0.965026681 | 0.005551587 | 0.965026681 |
| ENSG00000104904 | OAZ1 | 0.738696596 | 1.20419E-05 | 0.738696596 |
| ENSG00000106211 | HSPB1 | 1.387252913 | 0.00029292 | 1.387252913 |
| ENSG00000107281 | NPDC1 | -0.781810226 | 0.000157734 | 0.781810226 |
| ENSG00000107796 | ACTA2 | 1.162667148 | 0.000137547 | 1.162667148 |
| ENSG00000107862 | GBF1 | -0.710454707 | 0.017445717 | 0.710454707 |
| ENSG00000109971 | HSPA8 | 0.675012037 | 3.33441E-11 | 0.675012037 |
| ENSG00000110171 | TRIM3 | -0.697655294 | 0.000122493 | 0.697655294 |
| ENSG00000111077 | TNS2 | -0.590471079 | 0.020227182 | 0.590471079 |
| ENSG00000111252 | SH2B3 | -0.969136631 | 1.60892E-05 | 0.969136631 |
| ENSG00000111341 | MGP | 0.93002175 | 0.000632478 | 0.93002175 |
| ENSG00000111640 | GAPDH | 1.142742367 | 1.94777E-07 | 1.142742367 |
| ENSG00000111775 | COX6A1 | 0.628783189 | 1.26053E-08 | 0.628783189 |
| ENSG00000113140 | SPARC | 0.894404251 | 4.76476E-06 | 0.894404251 |
| ENSG00000117713 | ARID1A | -1.060278145 | 2.75276E-06 | 1.060278145 |
| ENSG00000120738 | EGR1 | -1.00349873 | 0.006360688 | 1.00349873 |
| ENSG00000120885 | CLU | 2.757243821 | 4.42145E-09 | 2.757243821 |
| ENSG00000123358 | NR4A1 | -5.065558143 | 4.05657E-08 | 5.065558143 |
| ENSG00000123416 | TUBA1B | 0.837378147 | 1.41634E-09 | 0.837378147 |
| ENSG00000125356 | NDUFA1 | 0.799519243 | 1.89763E-07 | 0.799519243 |
| ENSG00000125730 | C3 | 1.092154338 | 4.1011E-06 | 1.092154338 |
| ENSG00000125740 | FOSB | -1.905977274 | 0.011981582 | 1.905977274 |
| ENSG00000128016 | ZFP36 | -2.141798687 | 3.02412E-05 | 2.141798687 |
| ENSG00000131037 | EPS8L1 | -0.878979093 | 0.001374791 | 0.878979093 |
| ENSG00000131095 | GFAP | 3.726247809 | 8.48121E-05 | 3.726247809 |
| ENSG00000132470 | ITGB4 | -0.664021083 | 0.002561927 | 0.664021083 |
| ENSG00000132475 | H3-3B | 1.001205678 | 7.65756E-05 | 1.001205678 |
| ENSG00000133112 | TPT1 | 1.307510166 | 0.00045671 | 1.307510166 |
| ENSG00000133142 | TCEAL4 | 0.817810437 | 0.005433495 | 0.817810437 |
| ENSG00000133250 | ZNF414 | -1.395045216 | 0.002066892 | 1.395045216 |
| ENSG00000135404 | CD63 | 0.585329444 | 1.74459E-07 | 0.585329444 |
| ENSG00000135486 | HNRNPA1 | 1.539896541 | 4.3155E-05 | 1.539896541 |
| ENSG00000136156 | ITM2B | 0.629611282 | 0.001987625 | 0.629611282 |
| ENSG00000140400 | MAN2C1 | -0.615056332 | 0.005268865 | 0.615056332 |
| ENSG00000142156 | COL6A1 | -0.678023497 | 0.001787911 | 0.678023497 |
| ENSG00000142515 | KLK3 | -10.47763506 | 0.000764483 | 10.47763506 |
| ENSG00000142599 | RERE | -0.870419942 | 9.36718E-07 | 0.870419942 |
| ENSG00000146067 | FAM193B | -0.614647822 | 0.012923232 | 0.614647822 |
| ENSG00000146205 | ANO7 | -1.614052854 | 5.37859E-10 | 1.614052854 |
| ENSG00000149806 | FAU | 0.775357805 | 3.30801E-08 | 0.775357805 |
| ENSG00000149925 | ALDOA | 1.015674692 | 4.37283E-07 | 1.015674692 |
| ENSG00000156508 | EEF1A1 | 1.655187594 | 1.78296E-06 | 1.655187594 |
| ENSG00000158715 | SLC45A3 | -1.809476013 | 2.57772E-05 | 1.809476013 |
| ENSG00000159674 | SPON2 | -2.372862833 | 0.007712396 | 2.372862833 |
| ENSG00000163041 | H3-3A | 0.635811514 | 0.004439481 | 0.635811514 |
| ENSG00000164692 | COL1A2 | 0.72387276 | 2.36013E-10 | 0.72387276 |
| ENSG00000166165 | CKB | 1.042867722 | 0.001314876 | 1.042867722 |
| ENSG00000166444 | DENND2B | -0.656626111 | 0.012191165 | 0.656626111 |
| ENSG00000166710 | B2M | 2.539794721 | 0.019005831 | 2.539794721 |
| ENSG00000167615 | LENG8 | -1.078858291 | 1.90007E-07 | 1.078858291 |
| ENSG00000167642 | SPINT2 | 0.589541991 | 0.000781566 | 0.589541991 |
| ENSG00000167658 | EEF2 | 2.206379346 | 0.015963656 | 2.206379346 |
| ENSG00000167749 | KLK4 | -0.950662867 | 0.002670656 | 0.950662867 |
| ENSG00000167996 | FTH1 | 1.942634829 | 4.94462E-08 | 1.942634829 |
| ENSG00000169045 | HNRNPH1 | 1.930423183 | 7.37271E-05 | 1.930423183 |
| ENSG00000169926 | KLF13 | -0.645783959 | 0.00237095 | 0.645783959 |
| ENSG00000170345 | FOS | -2.579883551 | 0.002999296 | 2.579883551 |
| ENSG00000171223 | JUNB | -0.770672461 | 0.003509179 | 0.770672461 |
| ENSG00000177685 | CRACR2B | -0.786091989 | 2.27557E-05 | 0.786091989 |
| ENSG00000179094 | PER1 | -1.040199143 | 0.000317463 | 1.040199143 |
| ENSG00000181090 | EHMT1 | -0.661178077 | 0.000432913 | 0.661178077 |
| ENSG00000181163 | NPM1 | 0.807889668 | 0.002268178 | 0.807889668 |
| ENSG00000183889 | NPIPA6 | -0.593787646 | 0.048429486 | 0.593787646 |
| ENSG00000184009 | ACTG1 | 1.138450036 | 9.60393E-12 | 1.138450036 |
| ENSG00000184254 | ALDH1A3 | -1.496245435 | 2.27668E-08 | 1.496245435 |
| ENSG00000187244 | BCAM | -0.729233443 | 0.011793563 | 0.729233443 |
| ENSG00000188257 | PLA2G2A | 0.936413559 | 0.035384223 | 0.936413559 |
| ENSG00000196126 | HLA-DRB1 | 1.029210418 | 9.16255E-09 | 1.029210418 |
| ENSG00000196262 | PPIA | 1.453506375 | 6.82079E-14 | 1.453506375 |
| ENSG00000196498 | NCOR2 | -0.6124055 | 0.029215259 | 0.6124055 |
| ENSG00000197530 | MIB2 | -0.75408594 | 0.001383446 | 0.75408594 |
| ENSG00000197746 | PSAP | 0.605437589 | 5.69857E-05 | 0.605437589 |
| ENSG00000197903 | H2BC12 | 1.020213408 | 7.21446E-08 | 1.020213408 |
| ENSG00000197971 | MBP | 4.209281767 | 0.0001351 | 4.209281767 |
| ENSG00000204389 | HSPA1A | 0.651808715 | 0.006580941 | 0.651808715 |
| ENSG00000204628 | RACK1 | 0.685478577 | 0.030574479 | 0.685478577 |
| ENSG00000205542 | TMSB4X | 1.578452381 | 1.52324E-05 | 1.578452381 |
| ENSG00000233024 | NPIPA9 | -0.643579395 | 0.016880753 | 0.643579395 |
| ENSG00000240972 | MIF | 1.195129025 | 0.002609081 | 1.195129025 |
| ENSG00000246705 | H2AJ | -0.607599725 | 0.013435011 | 0.607599725 |
| ENSG00000250479 | CHCHD10 | 0.692358285 | 0.007590332 | 0.692358285 |
| ENSG00000251562 | MALAT1 | -1.069468512 | 0.004827387 | 1.069468512 |
| ENSG00000254772 | EEF1G | 0.941197815 | 7.8788E-05 | 0.941197815 |
| ENSG00000266714 | MYO15B | -1.56494463 | 9.48967E-06 | 1.56494463 |
| ENSG00000273542 | H4C12 | 0.773427785 | 8.7048E-07 | 0.773427785 |

### Screening and Analysis of Biomarkers Related to Prostate Cancer Metastasis Signaling Pathways

Pathway analysis was performed to determine the biological mechanisms associated with the biomarkers selected through the above screening. A list of signaling pathways containing keywords related to prostate cancer and major signaling pathways involved in cancer development was selected, and pathways associated with prostate cancer were identified from gene set enrichment analysis (GSEA) results based on genes showing differential expression.

In this process, the MSigDB database was used to extract gene sets included in the respective signaling pathways. To identify central genes exhibiting strong connectivity and high interaction levels within each signaling pathway, the STRING database, a protein-protein interaction database, was utilized. Based on the protein-protein interaction network, the top 10 central genes in each signaling pathway, which exhibited the highest centrality and connected the largest number of gene pairs, were selected, thereby identifying an additional 103 central genes.

As a result of the analysis, as shown in Table 2 below, 33 signaling pathways having the highest centrality among those closely associated with prostate cancer metastasis were identified, and combinations of biomarkers having the strongest associations within these signaling pathways were derived.

**[Table 2]**

| DB source | Signaling Pathway | Selected Genes |
|---|---|---|
| MSigDB | Prostate cancer | AKT1, CASP9, CCND1, CDK2, CDKN1A, CTNNB1, EP300, GRB2, GSK3B, HRAS, IKBKB, IKBKG, INS, KRAS, LEF1, MAPK1, MAPK3, MTOR, NFKB1, NFKBIA, NRAS, PIK3CA, PIK3R1, PTEN, RELA, SOS1, TP53 |
| MSigDB | Transcriptional misregulation in cancer | BCL2L1, CCNA2, CD40, CDKN1A, GADD45A, H3-3B, H3C12, H3C13, IL6, NFKB1, RELA, RUNX1, TP53 |
| MSigDB | TNF signaling pathway | AKT1, CASP3, CASP8, IKBKB, IKBKG, IL1B, IL6, JAG1, JUN, MAPK1, MAPK3, NFKB1, NFKBIA, PIK3CA, PIK3R1, RELA, TNF, TNFRSF1A |
| MSigDB | B cell receptor signaling pathway | AKT1, BTK, GRB2, GSK3B, HRAS, IKBKB, IKBKG, JUN, KRAS, LYN, MAPK1, MAPK3, NFKB1, NFKBIA, NRAS, PIK3CA, PIK3R1, PLCG2, RELA, SOS1, SYK |
| MSigDB | T cell receptor signaling pathway | AKT1, CDK4, FYN, GRB2, GSK3B, HRAS, IKBKB, IKBKG, JUN, KRAS, LCK, MAPK1, MAPK3, NFKB1, NFKBIA, NRAS, PIK3CA, PIK3R1, RELA, RHOA, SOS1, TNF |
| MSigDB | JAK-STAT signaling pathway | AKT1, BCL2L1, CCND1, CDKN1A, |
| | | EP300, GRB2, HRAS, IL6, JAK1, JAK2, JAK3, MTOR, PIK3CA, PIK3R1, SOS1, STAT1, STAT3, TYK2 |
| MSigDB | TGF-beta signaling pathway | ACVR1, BMP4, BMPR1A, EP300, MAPK1, MAPK3, RHOA, SMAD2, SMAD3, SMAD4, TFRC, TGFB1, TNF |
| MSigDB | Notch signaling pathway | DVL1, DVL2, EP300, JAG1, MAML1, MAML2, MAML3, NOTCH1, NOTCH2, NOTCH3, NOTCH4, RBPJ |
| MSigDB | Wnt signaling pathway | AXIN1, AXIN2, CCND1, CTNNB1, DVL1, DVL2, EP300, GSK3B, JUN, LEF1, RHOA, SMAD3, SMAD4, TP53 |
| MSigDB | Cellular senescence | AKT1, CCNA2, CCNB2, CCND1, CDK1, CDK2, CDK4, CDKN1A, CDKN2A, CHEK1, GADD45A, HRAS, IL6, KRAS, MAPK1, MAPK3, MTOR, NFKB1, NRAS, PIK3CA, PIK3R1, PTEN, RELA, RHEB, SMAD2, SMAD3, TGFB1, TP53, TSC2 |
| MSigDB | Necroptosis | CASP8, IL1B, JAK1, JAK2, JAK3, STAT1, STAT3, TNF, TNFRSF1A, TYK2 |
| MSigDB | Ferroptosis | ACSL4, GPX4, HMOX1, LPCAT3, NCOA4, TFRC, TP53 |
| MSigDB | Apoptosis I | ACTB, AKT1, BCL2L1, CASP3, CASP8, CASP9, GADD45A, HRAS, IKBKB, |
| | | IKBKG, JUN, KRAS, MAPK1, MAPK3, NFKB1, NFKBIA, NRAS, PIK3CA, PIK3R1, RELA, TNF, TNFRSF1A, TP53 |
| MSigDB | PI3K-Akt signaling pathway | AKT1, BCL2L1, BRCA1, CASP9, CCND1, CDK2, CDK4, CDKN1A, GNB1, GRB2, GSK3B, HRAS, IKBKB, IKBKG, IL6, INS, JAK1, JAK2, JAK3, KRAS, MAPK1, MAPK3, MLST8, MTOR, NFKB1, NRAS, PIK3CA, PIK3R1, PTEN, RELA, RHEB, RPTOR, SOS1, SYK, TP53, TSC2 |
| MSigDB | mTOR signaling pathway | AKT1, DVL1, DVL2, GRB2, GSK3B, HRAS, IKBKB, INS, KRAS, MAPK1, MAPK3, MLST8, MTOR, NRAS, PIK3CA, PIK3R1, PTEN, RHEB, RHOA, RPTOR, SOS1, TNF, TNFRSF1A, TSC2 |
| MSigDB | p53 signaling pathway | BCL2L1, CASP3, CASP8, CASP9, CCNB2, CCND1, CDK1, CDK2, CDK4, CDKN1A, CDKN2A, CHEK1, GADD45A, PTEN, TP53, TSC2 |
| MSigDB | Cell cycle | CCNA2, CCNB2, CCND1, CDK1, CDK2, CDK4, CDKN1A, CDKN2A, CHEK1, EP300, GADD45A, GSK3B, MCM7, SMAD2, SMAD3, SMAD4, TGFB1, TP53 |
| MSigDB | NF-kappa B signaling pathway | BCL2L1, BTK, CD40, GADD45A, IKBKB, IKBKG, IL1B, LCK, LYN, NFKB1, NFKBIA, PLCG2, RELA, SYK, TNF, TNFRSF1A, TRAF6 |
| MSigDB | Chemokine signaling pathway | AKT1, CCR5, CXCR4, GNB1, GRB2, GSK3B, HRAS, IKBKB, IKBKG, JAK2, JAK3, KRAS, LYN, MAPK1, MAPK3, NFKB1, NFKBIA, NRAS, PIK3CA, PIK3R1, PLCG2, RELA, RHOA, SOS1, SRC, STAT1, STAT3 |
| MSigDB | Rap1 signaling pathway | ACTB, AKT1, CTNNB1, HRAS, INS, KRAS, MAPK1, MAPK3, NRAS, PIK3CA, PIK3R1, RHOA, SRC |
| MSigDB | Ras signaling pathway | AKT1, BCL2L1, GNB1, GRB2, HRAS, IKBKB, IKBKG, INS, KRAS, MAPK1, MAPK3, NFKB1, NRAS, PIK3CA, PIK3R1, PLCG2, RELA, RHOA, SOS1 |
| MSigDB | ErbB signaling pathway | AKT1, CDKN1A, GRB2, GSK3B, HRAS, JUN, KRAS, MAPK1, MAPK3, MTOR, NRAS, PIK3CA, PIK3R1, PLCG2, SOS1, SRC |
| MSigDB | MAPK signaling pathway | AKT1, CASP3, GADD45A, GRB2, HRAS, IKBKB, IKBKG, IL1B, INS, JUN, KRAS, MAPK1, MAPK3, NFKB1, NRAS, RELA, SOS1, TGFB1, TNF, |
| | | TNFRSF1A, TP53, TRAF6 |
| MSigDB | WNT beta catenin signaling | AXIN1, AXIN2, CTNNB1, DVL2, JAG1, LEF1, MAML1, NOTCH1, NOTCH4, RBPJ, TP53 |
| MSigDB | NOTCH signaling | CCND1, DVL1, DVL2, EP300, JAG1, MAML1, MAML2, MAML3, NOTCH1, NOTCH2, NOTCH3, NOTCH4, RBPJ |
| MSigDB | Estrogen Response LATE | CCND1, JAK1, JAK2 |
| MSigDB | Estrogen Response EARLY | CCND1, JAK2 |
| MSigDB | E2F Targets | BRCA1, CCNB2, CDK1, CDK4, CDKN1A, CDKN2A, CHEK1, MCM7, TFRC, TP53 |
| MSigDB | Apoptosis II | BCL2L1, BRCA1, CASP3, CASP8, CASP9, CCND1, CDK2, CDKN1A, CTNNB1, GADD45A, GPX4, HMOX1, IL1B, IL6, JUN, LEF1, RELA, TNF |
| MSigDB | Adipogenesis | ACLY, ACOX1, CS, GADD45A, GPX4, LPCAT3, SDHB |
| MSigDB | ESR-mediated signaling | AKT1, AXIN1, CCND1, EP300, GNB1, H3-3B, H3C12, H3C13, H4C6, HRAS, JUN, KRAS, MAPK1, MAPK3, NRAS, PIK3CA, PIK3R1, RUNX1, SRC |
| MSigDB | Estrogen-dependent gene expression | AXIN1, CCND1, EP300, H3-3B, H3C12, H3C13, H4C6, JUN, RUNX1 |
| MSigDB | Androgen receptor network in prostate cancer | AKT1, BRCA1, CASP3, CASP8, CASP9, CCND1, CDK1, CDK2, CDK4, CHEK1, GRB2, HRAS, JAK1, JUN, MAPK1, MAPK3, MTOR, PIK3CA, PTEN, RHEB, RPTOR, SMAD2, SMAD3, SOS1, STAT1, STAT3, TP53, TSC2 |

### Selection of Final Biomarkers

The list of housekeeping genes was derived from the HRT Atlas v1.0 database, and only genes overlapping between the external public dataset (TCGA) and the in-house dataset were selected. This list extraction step was performed under appropriate experimental conditions and was conducted to provide supporting evidence of sample quality.

Genes exhibiting high expression levels were preferentially selected to minimize the influence of experimental noise and other factors while ensuring stable expression levels. In addition, gene-wise variability was analyzed to further select genes exhibiting low variability, thereby ensuring consistent expression patterns and reproducibility of experimental results. Genes satisfying both high expression and low variance criteria in each dataset were selected, and rank-based scores were assigned to 23 genes to select the top 10 reference genes.

The top 10 genes were identified based on statistical metrics, including minimum and maximum expression levels and coefficient of variation, and comprised PPP2R1A, LAMTOR1, RNF167, ENSA, AP2M1, RNF10, BANF1, SLC25A3, APH1A, and DNAJB2.

Finally, 103 genes exhibiting differential expression and 103 central genes selected from 33 signaling pathways were identified. Among these, H3-3B and ACTB overlapped; therefore, a total of 204 genes were derived as the final biomarker combination for diagnosing prostate cancer prognosis, metastasis, and/or recurrence.

### Validation of Prostate Cancer Prognosis Prediction

Clinical validation was performed on 172 prostate cancer patients with a follow-up period of 5 years or longer. Risk scores were calculated for all patients using the aforementioned prognostic prediction tool, and the performance was comparatively validated against existing products.

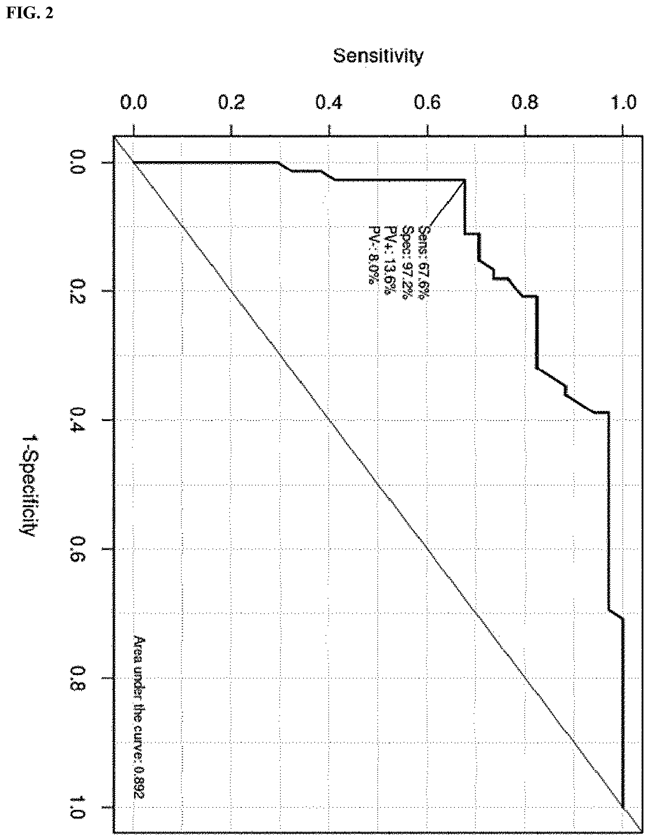

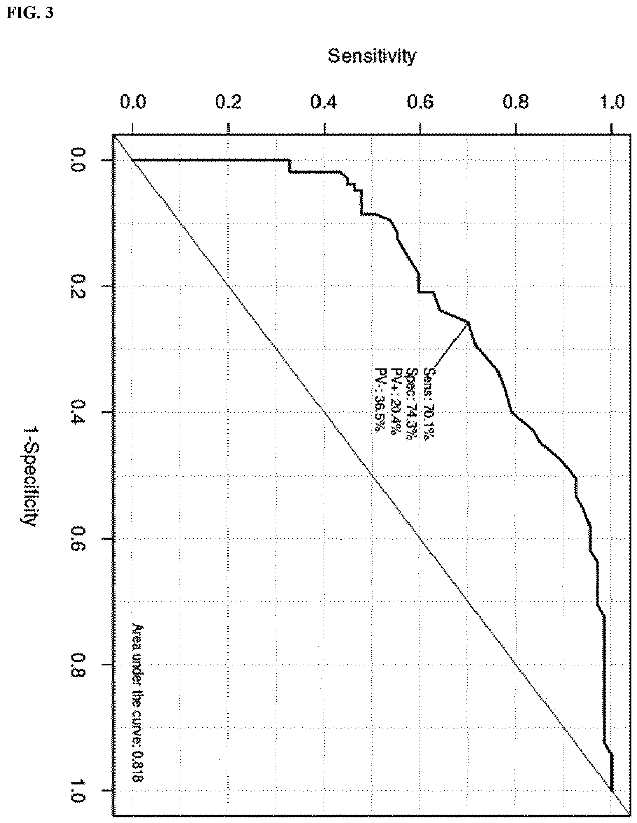

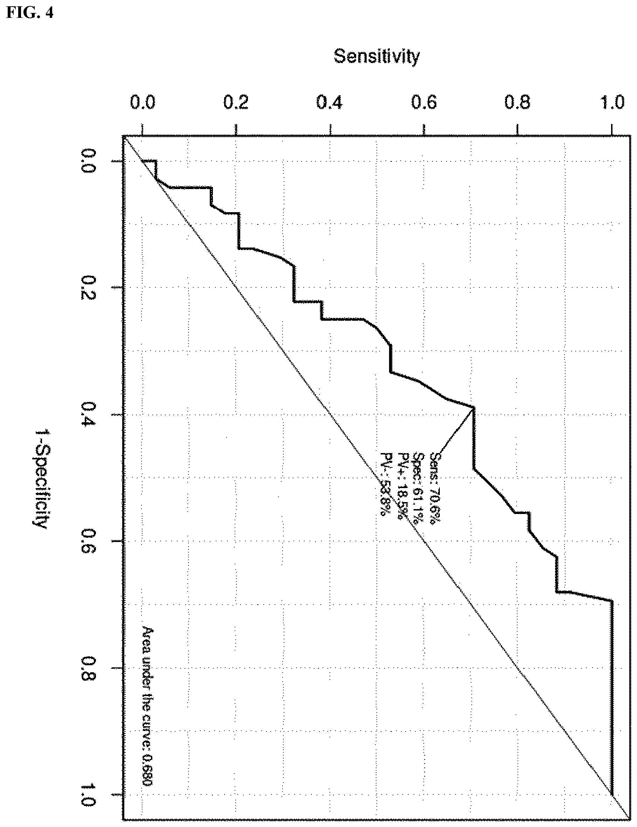

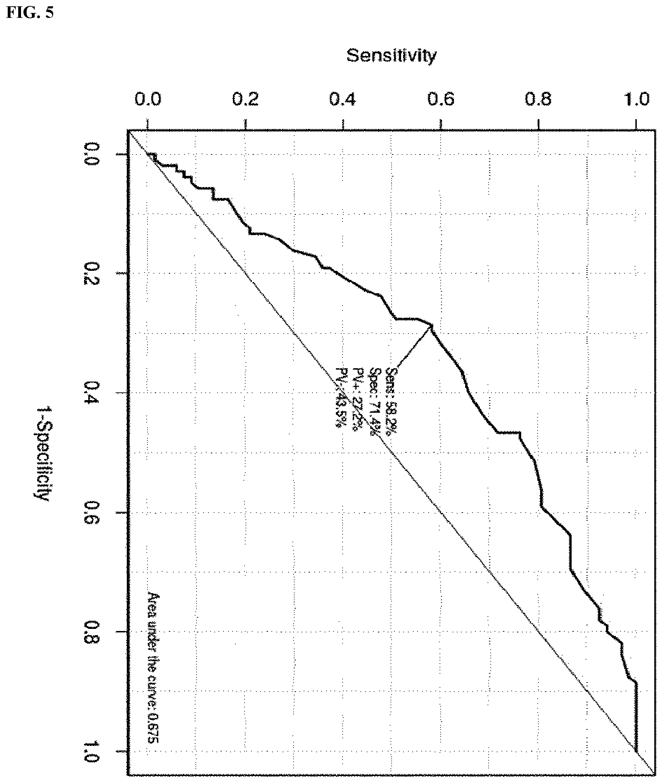

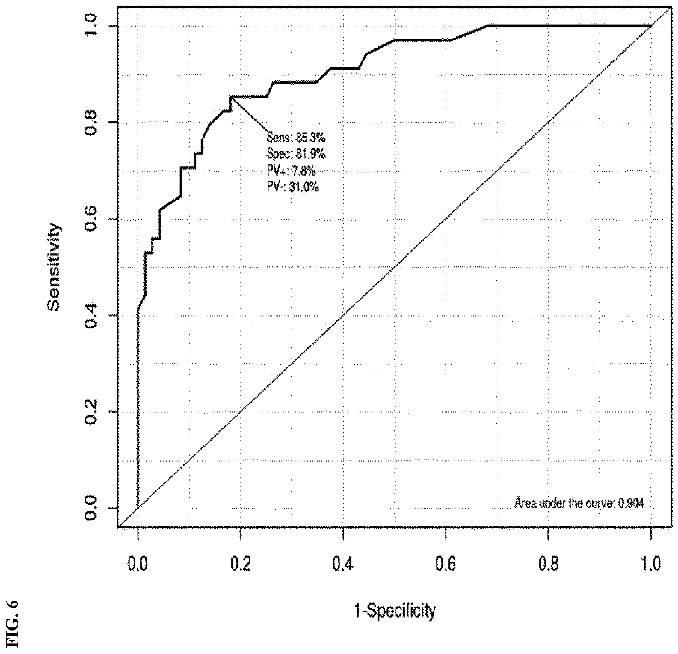

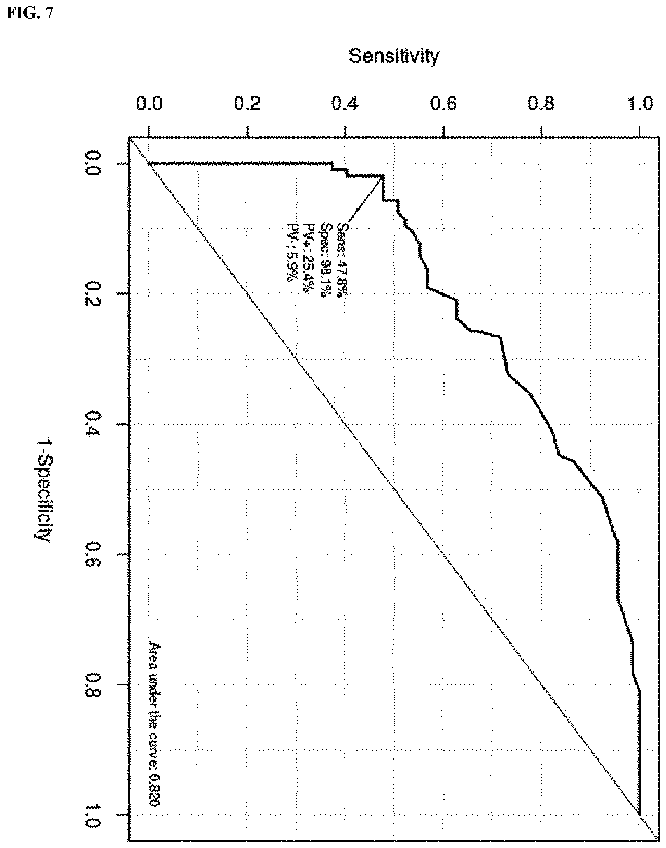

In order to improve predictive performance during the validation process, a Random Forest algorithm-based classification model was applied. Results obtained using only 103 differentially expressed gene (DEG) markers (ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414) (see FIGs. 2 and 3), results obtained using only signaling pathway-related markers (ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2) (see FIGs. 4 and 5), and results obtained using both DEG markers and signaling pathway-related markers in combination (see FIGs. 6 and 7) were evaluated.

Referring to FIG. 2, when DEG markers were used alone, the AUC value was 0.892, indicating excellent diagnostic performance. Referring to FIG. 6, when signaling pathway-related markers were applied in combination with DEG markers, the AUC value increased to 0.904, demonstrating a significant improvement in model performance (see Table 3).

**[Table 3]**

| **Feature Set** | **Dataset** | **Sensitivit y** | **Specificit y** | **PPV** | **NPV** | **F1** | **Accurac y** | **AUC** |
|---|---|---|---|---|---|---|---|---|
| **DEG + pathway** | RF_testS et | 0.8529 | 0.8194 | 0.6905 | 0.9219 | 0.7632 | 0.8302 | 0.904 |
| | RF_valid Set | 0.5672 | 0.8095 | 0.6552 | 0.7456 | 0.608 | 0.7151 | 0.8203 |
| **DEG** | RF_testS et | 0.6765 | 0.9722 | 0.92 | 0.8642 | 0.7797 | 0.8774 | 0.8922 |
| | RF_valid Set | 0.4627 | 0.9524 | 0.8611 | 0.7353 | 0.6019 | 0.7616 | 0.8183 |
| **Pathway** | RF_testS et | 0.7059 | 0.6111 | 0.4615 | 0.8148 | 0.5581 | 0.6415 | 0.6801 |
| | RF_valid Set | 0.4478 | 0.7714 | 0.5556 | 0.6864 | 0.4959 | 0.6453 | 0.6748 |
| | | | | | | | | |

As shown in the above results, when a combination of the 103 DEG markers and 103 signaling pathway-related markers of the present invention is used, it is possible to overcome the limitations of conventional prostate cancer diagnostic models and to more precisely predict prostate cancer prognosis, as well as the likelihood of metastasis and recurrence. Based on these findings, the present invention is expected to significantly contribute to improving the survival rate of prostate cancer patients.

### Preprocessing of Input Data for the Prostate Cancer Risk and Prognosis Prediction Algorithm

The 103 DEG markers and 33 signaling pathways derived above were used as explanatory variables, specifically the 103 gene markers listed in Table 1 and the 33 signaling pathway-related gene markers listed in Table 2. Event information was used as the response variable, and initial data preprocessing was performed by excluding missing values.

The list of genes showing differential expression was obtained using log2(TPM)-transformed data to reflect differences in gene expression between patients with metastasis and those without. The list of core genes (signaling pathway-related genes) was used in ssGSVA analysis to calculate a score representing the activation level of each signaling pathway for each sample. A positive score indicates that the corresponding pathway is highly activated, whereas a negative score indicates that the pathway is relatively inactive.

The calculated data were normalized to the log2(TPM) scale to enable analysis on a common scale with gene expression levels. Based on the selected biomarkers, the correlation between follow-up duration and events (metastasis, BCR, and OS) was analyzed, and Cox regression analysis was performed to estimate factors influencing time-to-event.

### Cox Regression Analysis Algorithm

The regression coefficients estimated by Cox regression analysis represent the influence of each explanatory variable on survival time. Based on this, a patient-specific risk score was calculated by summing the products of the values of the explanatory variables (differentially expressed gene expression levels and signaling pathway scores) and their corresponding Cox regression coefficients.

The performance of the model was evaluated with respect to its ability to calculate patient-specific risk scores using the estimated regression coefficients and the values of each explanatory variable, and to determine the presence or absence of metastasis based on the calculated scores. In addition, the risk level and risk classification of each variable were predicted based on the regression coefficient values.

This relationship can be mathematically expressed as Equation 1 below, where p represents the number of explanatory variables.
$Risk Score = {\sum }_{i = 1}^{p} Regression {Coefficients}_{i} \times Explanatory {Variables}_{i}$
he regression coefficients estimated for each gene based on the regression analysis are shown in Table 4, and the regression coefficients estimated for each signaling pathway are shown in Table 5. The final risk score was calculated as the sum of the values in the "each term of the risk score" column in Tables 4 and 5.

In the "each term of the risk score," the gene name corresponds to the expression level of each gene, and the signaling pathway name corresponds to the signaling pathway score. The signaling pathway score is calculated using GSVA (Gene Set Variation Analysis) based on the signaling pathway-related genes included in each signaling pathway and listed in Table 2.

Table 4 shows the expression levels of each gene as explanatory variables, corresponding to the 103 differentially expressed gene (DEG) markers identified above, and Table 5 shows 33 signaling pathway scores, as explanatory variables, calculated from the signaling pathway-related genes.

**[Table 4]**

| **Explanatory Variables** | **Regression Coefficient** | **Terms of the Risk Score** |
|---|---|---|
| EEF1A1 | -24.5157 | -24.5157 * EEF1A1 |
| PKM | -20.6784 | -20.6784 * PKM |
| CST3 | -20.6048 | -20.6048 * CST3 |
| RACK1 | -19.7729 | -19.7729 * RACK1 |
| HSPA8 | -16.1424 | -16.1424 * HSPA8 |
| FTH1 | -14.4929 | -14.4929 * FTH1 |
| ARID1A | -10.9563 | -10.9563 * ARID1A |
| SLC45A3 | -10.8547 | -10.8547 * SLC45A3 |
| TPT1 | -10.3515 | -10.3515 * TPT1 |
| SPARC | -9.7075 | -9.7075 * SPARC |
| NCOR2 | -9.6148 | -9.6148 * NCOR2 |
| RERE | -8.7200 | -8.72 * RERE |
| ALDH1A3 | -8.1487 | -8.1487 * ALDH1A3 |
| ACTA2 | -7.9225 | -7.9225 * ACTA2 |
| EGR1 | -7.5508 | -7.5508 * EGR1 |
| TCEAL4 | -7.0247 | -7.0247 * TCEAL4 |
| MYL6 | -6.4505 | -6.4505 * MYL6 |
| HSF4 | -6.1940 | -6.194 * HSF4 |
| EHMT1 | -5.9082 | -5.9082 * EHMT1 |
| MYO15B | -5.7667 | -5.7667 * MYO15B |
| COX6A1 | -5.6728 | -5.6728 * COX6A1 |
| EEF1G | -4.6997 | -4.6997 * EEF1G |
| H3-3B | -4.3425 | -4.3425 * H3-3B |
| DENND2B | -4.1949 | -4.1949 * DENND2B |
| PLA2G2A | -4.0087 | -4.0087 * PLA2G2A |
| JUNB | -3.8773 | -3.8773 * JUNB |
| TNS2 | -3.8637 | -3.8637 * TNS2 |
| MAN2C1 | -3.8205 | -3.8205 * MAN2C1 |
| NDRG1 | -3.7061 | -3.7061 * NDRG1 |
| SH2B3 | -3.5983 | -3.5983 * SH2B3 |
| GSTP1 | -3.5252 | -3.5252 * GSTP1 |
| TNK2 | -3.1408 | -3.1408 * TNK2 |
| NR4A1 | -3.0147 | -3.0147 * NR4A1 |
| C3 | -3.0057 | -3.0057 * C3 |
| ITM2B | -3.0036 | -3.0036 * ITM2B |
| HNRNPH1 | -2.9322 | -2.9322 * HNRNPH1 |
| PER1 | -2.7527 | -2.7527 * PER1 |
| ZNF414 | -2.2967 | -2.2967 * ZNF414 |
| HLA-DRB1 | -2.2345 | -2.2345 * HLA-DRB1 |
| MBP | -2.1829 | -2.1829 * MBP |
| NPIPA9 | -2.1081 | -2.1081 * NPIPA9 |
| OAZ1 | -1.8119 | -1.8119 * OAZ1 |
| SPON2 | -1.5800 | -1.58 * SPON2 |
| ITGB4 | -1.2803 | -1.2803 * ITGB4 |
| SPINT2 | -1.1729 | -1.1729 * SPINT2 |
| MIF | -1.0697 | -1.0697 * MIF |
| MALAT1 | -0.8059 | -0.8059 * MALAT1 |
| ZFP36 | -0.7027 | -0.7027 * ZFP36 |
| PKD1 | -0.5050 | -0.505 * PKD1 |
| H4C12 | -0.2988 | -0.2988 * H4C12 |
| SREBF 1 | 0.3901 | 0.3901 * SREBF1 |
| COL1A2 | 0.5308 | 0.5308 * COL1A2 |
| HSPB1 | 0.6278 | 0.6278 * HSPB1 |
| TMSB4X | 0.6909 | 0.6909 * TMSB4X |
| KLF 13 | 0.8907 | 0.8907 * KLF13 |
| FOSB | 0.9892 | 0.9892 * FOSB |
| ACTG1 | 1.4413 | 1.4413 * ACTG1 |
| GFAP | 1.4805 | 1.4805 * GFAP |
| GBF1 | 1.7382 | 1.7382 * GBF1 |
| WDR1 | 1.8334 | 1.8334 * WDR1 |
| H2AJ | 1.8547 | 1.8547 * H2AJ |
| H2BC12 | 2.0712 | 2.0712 * H2BC12 |
| EPS8L1 | 2.0753 | 2.0753 * EPS8L1 |
| CLU | 2.3705 | 2.3705 * CLU |
| GAPDH | 2.4415 | 2.4415 * GAPDH |
| MIB2 | 2.8416 | 2.8416 * MIB2 |
| NPM1 | 3.0533 | 3.0533 * NPM1 |
| NDUFA1 | 3.0995 | 3.0995 * NDUFA1 |
| ACTB | 3.3435 | 3.3435 * ACTB |
| TUBA1B | 3.3650 | 3.365 * TUBA1B |
| CHCHD10 | 3.4110 | 3.411 * CHCHD10 |
| TRIM3 | 3.6263 | 3.6263 * TRIM3 |
| FTL | 4.0749 | 4.0749 * FTL |
| CD63 | 4.1198 | 4.1198 * CD63 |
| KLK4 | 4.1571 | 4.1571 * KLK4 |
| HSPB6 | 4.2707 | 4.2707 * HSPB6 |
| EEF2 | 4.4645 | 4.4645 * EEF2 |
| TMSB10 | 4.5123 | 4.5123 * TMSB10 |
| DDT | 4.5437 | 4.5437 * DDT |
| H3-3A | 4.5734 | 4.5734 * H3-3A |
| FAM193B | 4.7982 | 4.7982 * FAM193B |
| HSPA1A | 4.9058 | 4.9058 * HSPA1A |
| NPDC1 | 5.3945 | 5.3945 * NPDC1 |
| PSAP | 5.4786 | 5.4786 * PSAP |
| CKB | 5.6318 | 5.6318 * CKB |
| CRACR2B | 5.7943 | 5.7943 * CRACR2B |
| ANO7 | 6.6179 | 6.6179 * ANO7 |
| KLK3 | 6.8742 | 6.8742 * KLK3 |
| LTBP4 | 6.9151 | 6.9151 * LTBP4 |
| NPIPA6 | 8.2184 | 8.2184 * NPIPA6 |
| PPIA | 8.6102 | 8.6102 * PPIA |
| LENG8 | 8.7982 | 8.7982 * LENG8 |
| MGP | 8.9408 | 8.9408 * MGP |
| ENO1 | 8.9458 | 8.9458 * ENO1 |
| PEBP1 | 9.2608 | 9.2608 * PEBP1 |
| B2M | 9.5207 | 9.5207 * B2M |
| FOS | 11.2197 | 11.2197 * FOS |
| HNRNPC | 12.2758 | 12.2758 * HNRNPC |
| BCAM | 12.2843 | 12.2843 * BCAM |
| FAU | 13.6036 | 13.6036 * FAU |
| COL6A1 | 13.7553 | 13.7553 * COL6A1 |
| HNRNPA1 | 13.7919 | 13.7919 * HNRNPA1 |
| ALDOA | 19.5991 | 19.5991 * ALDOA |

**[Table 5]**

| **Explanatory Variables** | **Regression Coefficient** | **Each component of the Risk Score** |
|---|---|---|
| p53_signaling_pathway | -12.2718 | -12.2718 * p53_signaling_pathway |
| Chemokine _signaling_pathway | -11.3817 | -11.3817 * Chemokine_signaling_pathway |
| ESR-MEDIATED SIGNALING | -11.3383 | -11.3383 * ESR-MEDIATED _SIGNALING |
| B-cell receptor signaling pathway | -9.4816 | -9.4816 * B-cell receptor signaling |
| | | pathway |
| mTOR signaling pathway | -8.1686 | -8.1686 * mTOR signaling pathway |
| Wnt signaling pathway | -7.7882 | -7.7882 * Wnt_signaling_pathway |
| TNF signaling pathway | -5.8849 | -5.8849 * TNF signaling pathway |
| APOPTOSIS II | -2.4220 | -2.422 * APOPTOSIS II |
| NOTCH_SIGNALING | -1.5922 | -1.5922 * NOTCH_SIGNALING |
| Prostate_cancer | -1.4705 | -1.4705 * Prostate_cancer |
| Androgen_receptor_network_in_prost ate_cancer | -1.2326 | -1.2326 * Androgen_Receptor_Network_in_Prostate _Cancer |
| Ferroptosis | -0.9126 | -0.9126 * Ferroptosis |
| E2F_TARGETS | -0.4964 | -0.4964 * E2F_TARGETS |
| WNT_BETA_CATENIN_SIGNALIN G | -0.1242 | -0.1242 * WNT_BETA_CATENIN_SIGNALING |
| ESTROGEN_RESPONSE_EARLY | -0.0156 | -0.0156 * ESTROGEN_RESPONSE_EARLY |
| MAPK_signaling_pathway | 0.4950 | 0.495 * MAPK_signaling_pathway |
| Apoptosis I | 1.3209 | 1.3209 * Apoptosis I |
| ESTROGEN_DEPENDENT_GENE_ EXPRESSION | 1.3557 | 1.3557 * ESTROGEN_DEPENDENT_GENE_EXP RESSION |
| NF-κB signaling pathway | 1.5011 | 1.5011 * NF-κB signaling pathway |
| Rap1 signaling pathway | 1.5930 | 1.593 * Rap1_signaling_pathway |
| Necroptosis | 1.5948 | 1.5948 * Necroptosis |
| Cell_cycle | 1.6349 | 1.6349 * Cell_cycle |
| TGF-beta signaling pathway | 1.7569 | 1.7569 * TGF-beta signaling pathway |
| JAK-STAT signaling pathway | 1.9388 | 1.9388 * JAK-STAT signaling pathway |
| ESTROGEN_RESPONSE_LATE | 2.2348 | 2.2348 * ESTROGEN_RESPONSE_LATE |
| ADIPOGENESIS | 2.6037 | 2.6037 * ADIPOGENESIS |
| Transcriptional_misregulation_in_canc er | 2.6746 | 2.6746 * Transcriptional _misregulation_in_cancer |
| PI3K-Akt signaling pathway | 3.6056 | 3.6056 * PI3K-Akt signaling pathway |
| Notch signaling pathway | 5.8037 | 5.8037 * Notch_signaling_pathway |
| Cellular senescence | 7.7121 | 7.7121 * Cellular_senescence |
| ErbB signaling pathway | 9.3989 | 9.3989 * ErbB_signaling_pathway |
| Ras signaling pathway | 10.0083 | 10.0083 * Ras_signaling_pathway |
| T-cell receptor signaling pathway | 11.6220 | 11.622 * T-cell_receptor_signaling_pathway |

### Sample classifying based on risk score

A receiver operating characteristic (ROC) curve was generated to evaluate the likelihood of metastasis in patients based on the risk scores calculated from the Cox regression analysis. The ROC curve is used to assess model performance by plotting the true positive rate against the false positive rate at different thresholds, and the area under the ROC curve (AUC) indicates predictive performance, with values closer to 1 representing higher accuracy.

The optimal cutoff value of -257.50477 was determined based on the point at which the AUC was maximized. Using this cutoff value, patients with a risk score of -257.50477 or higher were classified into a High group, whereas those with a risk score below -257.50477 were classified into a Low group.

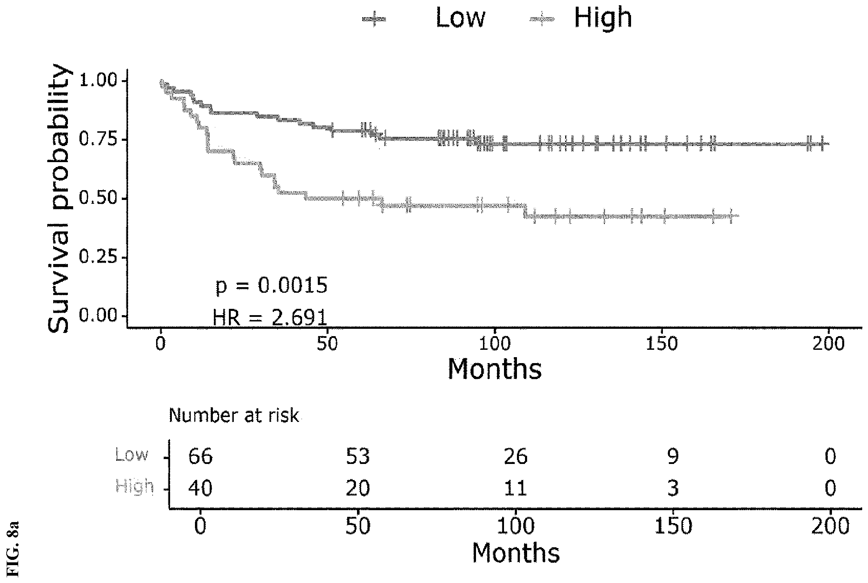

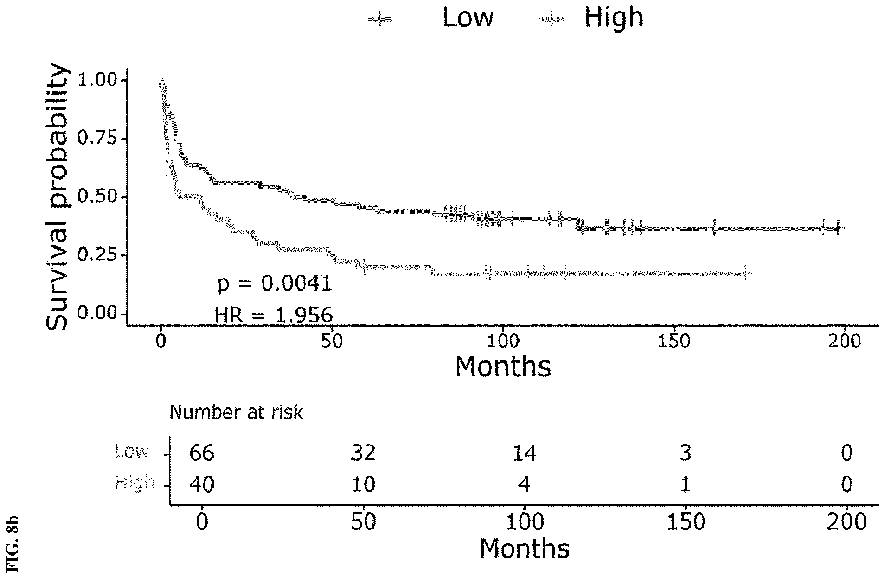

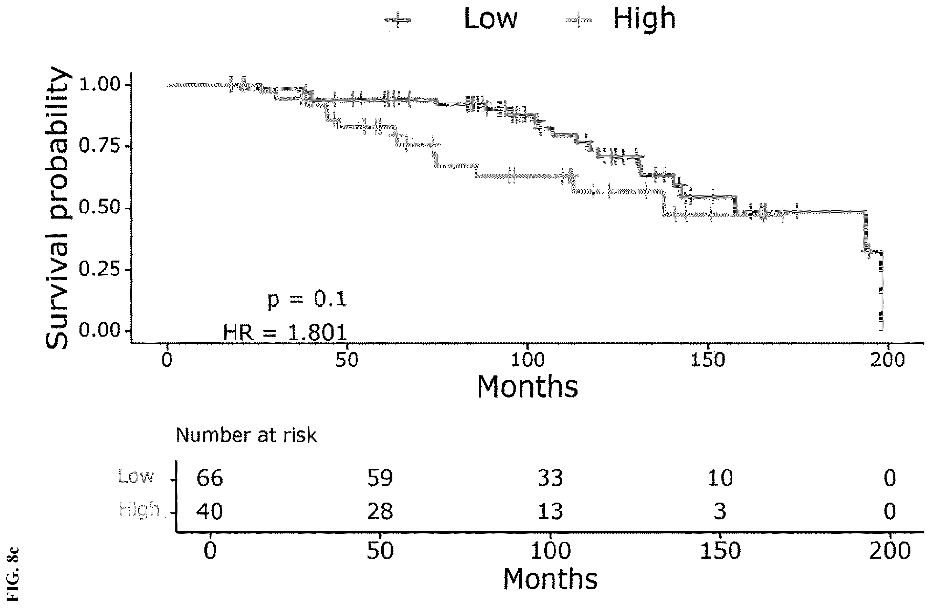

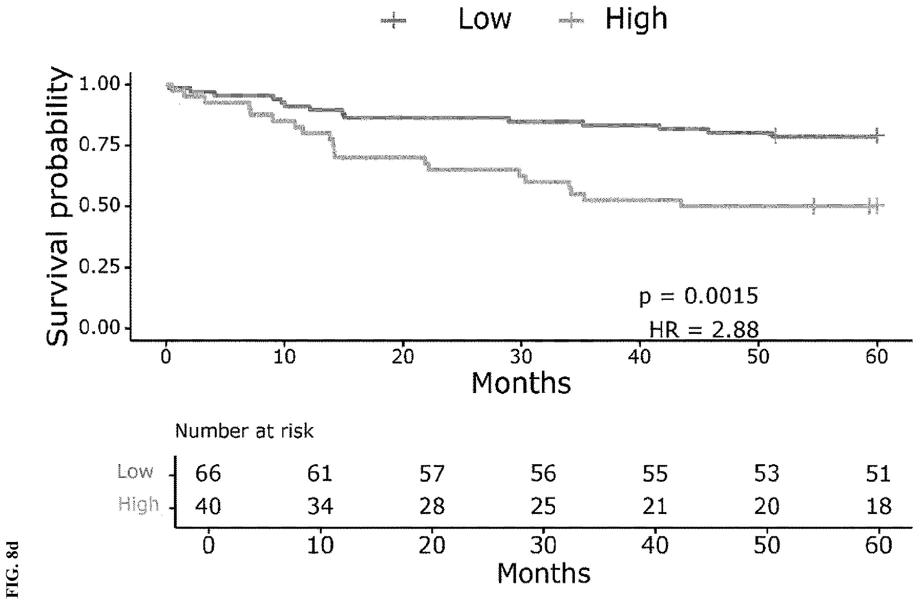

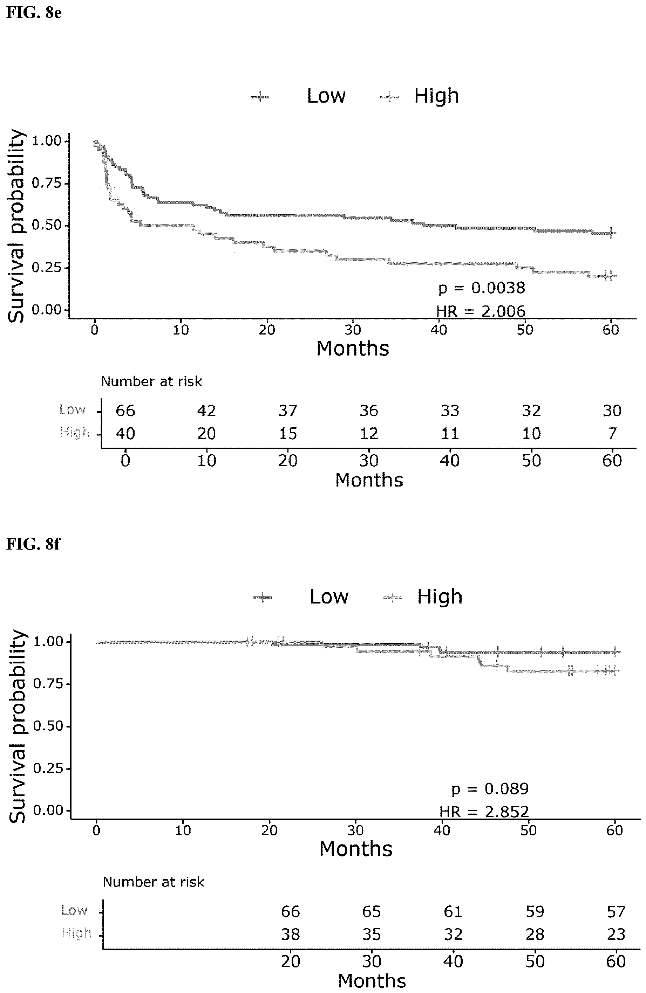

Differences in the distributions of survival, metastasis, and biochemical recurrence between the groups were assessed using Kaplan-Meier survival analysis and the log-rank test, as shown in Table 6 and FIGs. 8a to 8f.

The results showed that, in the Cox model, the distributions of metastasis and biochemical recurrence exhibited p-values < 0.05, indicating statistically significant differences. Such significance was observed not only over the entire follow-up period but also in 5-year metastasis-free survival and 5-year biochemical recurrence-free survival.

However, since the cutoff value was derived from a model designed to determine the presence of metastasis, differences in overall survival distributions were not statistically significant (p-value > 0.05) regardless of the follow-up period. Nevertheless, the hazard ratio (HR) indicated that the High group was associated with a higher risk compared to the Low group.

**[Table 6]**

| Follow-up | Survival Type | P-value | HR (Hazard Ratio) |
|---|---|---|---|
| Complete Follow-up | MFS | p = 0.0015 | 2.691 |
| | BFS | p = 0.0041 | 1.956 |
| | OS | P = 0.1 | 1.801 |
| 5-year follow-up | MFS | p = 0.0015 | 2.88 |
| | BFS | p = 0.0038 | 2.006 |
| | OS | P=0.089 | 2.852 |
| | | | |

### Validation of prostate cancer prognosis prediction using the algorithm

We further validated the ability of a Cox multivariate regression model, which combines the expression levels of 103 differentially expressed genes (DEGs) and the activation scores of 33 signaling pathways, to accurately predict prostate cancer metastasis based on the calculated risk scores.

As a result of prostate cancer metastasis prediction using the Cox regression model, an AUC of 0.632 and an accuracy (ACC) of 0.6792 were obtained. The ROC curve is illustrated in FIG. 3.

Based on these results, it was confirmed that the present invention can provide information on prostate cancer risk and prognosis using the risk scores derived from the combined application of DEG markers and signaling pathway-related markers. It is expected that early prediction of metastasis using this approach will significantly contribute to improving the survival rate of prostate cancer patients.

While specific embodiments of the present invention have been described in detail above, it will be apparent to those skilled in the art that such embodiments are provided for illustrative purposes only and do not limit the scope of the present invention. Accordingly, the scope of the present invention should be defined by the appended claims and their equivalents.

## Claims

1. A method for providing information on cancer risk, the method comprising:
(a) calculating gene expression levels and signaling pathway scores for a biological sample obtained from a subject and classifying them as explanatory variables;
(b) calculating a risk score of Equation 1 using a Cox regression analysis algorithm based on a combination of at least one gene listed in Table 1 and at least one signaling pathway listed in Table 2; and
(c) classifying the biological sample based on the calculated risk score; $Risk Score = {\sum }_{i = 1}^{p} Regression {Coefficients}_{i} \times Explanatory {Variables}_{i}$
wherein, in Equation 1,
p represents the number of explanatory variables, and the explanatory variables represent gene expression levels or signaling pathway scores.

2. The method of claim 1, wherein the gene is at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414.

3. The method of claim 1, wherein the signaling pathway is at least one signaling pathway selected from the group consisting of adipogenesis pathway, apoptosis pathway II, E2F targets pathway, estrogen response early pathway, estrogen response late pathway, NOTCH signaling pathway, WNT beta catenin signaling pathway, MAPK signaling pathway, ErbB signaling pathway, Ras signaling pathway, Rap1 signaling pathway, chemokine signaling pathway, NF-kappa B signaling pathway, cell cycle pathway, p53 signaling pathway, mTOR signaling pathway, PI3K-Akt signaling pathway, apoptosis pathway I, ferroptosis pathway, necroptosis pathway, cellular senescence pathway, Wnt signaling pathway, Notch signaling pathway, TGF-beta signaling pathway, JAK-STAT signaling pathway, T cell receptor signaling pathway, B cell receptor signaling pathway, TNF signaling pathway, transcriptional misregulation pathway in cancer, prostate cancer pathway, ESR-mediated signaling pathway, estrogen-dependent gene expression pathway, and androgen receptor network pathway in prostate cancer.

4. The method of claim 1, wherein the signaling pathway score is calculated based on the signaling pathway-related genes listed in Table 2 for each signaling pathway, using a GSVA (Gene Set Variation Analysis) program.

5. The method of claim 4, wherein the signaling pathway-related gene is at least one gene selected from the group consisting of ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2.

6. The method of claim 1, wherein the regression coefficients in Equation 1 are within a ±5% of the respective regression coefficient values corresponding to each explanatory variable listed in Table 4 or Table 5.

7. The method of claim 1, wherein the classifying comprises dividing the biological sample into a High group and a Low group based on a cutoff value of the risk score obtained by multiplying respective regression coefficients corresponding to each explanatory variable by a variable-specific value.

8. The method of claim 7, wherein the variable-specific value is a gene expression level of a gene listed in Table 1 or a signaling pathway score derived from signaling pathway-related genes listed in Table 2.

9. The method of claim 7, wherein the High group comprises subjects having a high likelihood of cancer metastasis or recurrence.

10. The method of claim 7, wherein the Low group comprises subjects having a low likelihood of cancer metastasis or recurrence.

11. The method of claim 1, wherein the cancer is at least one cancer selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, endocervical cancer, fallopian tube cancer, vaginal cancer, ovarian cancer, melanoma, skin cancer, hematologic cancer, bone cancer, brain cancer, endocrine cancer, parathyroid cancer, ureteral cancer, urethral cancer, bronchial cancer, bladder cancer, bone marrow cancer, leukemia, brain tumor, intestinal cancer, esophageal cancer, Ewing's sarcoma, tongue cancer, lymphoma, Kaposi's sarcoma, mesothelioma, multiple myeloma, neuroblastoma, osteosarcoma, and retinoblastoma.

12. A diagnostic device for predicting cancer risk, comprising:
(a) an input unit configured to receive gene expression levels and signaling pathway scores for a biological sample obtained from a subject;
(b) a processing unit configured to calculate a risk score according to Equation 1 using a Cox regression analysis algorithm based on a combination of at least one gene listed in Table 1 and at least one signaling pathway listed in Table 2; and
(c) an output unit configured to classify the biological sample into a High group or a Low group based on the calculated risk score value, and to output the classification result; $Risk Score = {\sum }_{i = 1}^{p} Regression {Coefficients}_{i} \times Explanatory {Variables}_{i}$
wherein, in Equation 1,
p represents the number of explanatory variables, and the explanatory variables represent gene expression levels or signaling pathway scores.
